(19) 

**Europäisches Patentamt**
**European Patent Office**
**Office européen des brevets**

(11)  **EP 3 127 472 B1**

(12)  **EUROPEAN PATENT SPECIFICATION**

(45)  Date of publication and mention
of the grant of the patent:
**09.10.2019  Bulletin 2019/41**

(51)  Int Cl.:
**A61B 3/10** *(2006.01)*  **A61B 3/12** *(2006.01)*
**G01B 9/02** *(2006.01)*

(21)  Application number: **15180259.2**

(22)  Date of filing: **07.08.2015**

(54)  **METHOD AND PROGRAM FOR POSITIONING AN MAGE OF AN OBJECT ON A TOMOGRAM AND AN OPTICAL COHERENCE TOMOGRAPHY APPARATUS THEREFOR**

VERFAHREN UND PROGRAMM ZUR POSITIONIERUNG EINES BILDES EINES OBJEKTS AUF EINEM TOMOGRAMM UND OPTISCHE KOHÄRENZTOMOGRAFIEVORRICHTUNG DAFÜR

PROCÉDÉ ET PROGRAMME POUR POSITIONNER UNE IMAGE D'UN OBJET SUR UN TOMOGRAMME ET APPAREIL DE TOMOGRAPHIE PAR COHÉRENCE OPTIQUE CORRESPONDANT

(84)  Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43)  Date of publication of application:
**08.02.2017  Bulletin 2017/06**

(73)  Proprietor: **CANON KABUSHIKI KAISHA Ohta-ku Tokyo 146-8501 (JP)**

(72)  Inventors:
• **DZIUBAK, Tomasz**
  **87-100 Torun (PL)**

• **BAJRASZEWSKI, Tomasz**
  **87-123 Glogowo (PL)**

(74)  Representative: **Garner, Jonathan Charles Stapleton Canon Europe Ltd European Patent Department 3 The Square Stockley Park Uxbridge, Middlesex UB11 1ET (GB)**

(56)  References cited:
**US-A1- 2009 244 547    US-A1- 2010 014 089 US-A1- 2011 181 889**

EP 3 127 472 B1

Description

Technical field

The present invention relates to the field of optical coherence tomography (OCT), in particular the medical area of ophthalmology, for which there is a demand for viewing the image of an object on a tomographic image. In particular, the present invention relates to a method and program for positioning of an image of an object on a tomogram and an optical coherence tomography apparatus therefor.

Background

Currently, there are various types of ophthalmological devices which use optical instruments in order to view an eye, such as an anterior ocular segment imaging instrument, a fundus camera, a confocal laser scanning ophthalmoscope (scanning laser ophthalmoscope: SLO), and the like. In particular, an imaging apparatus, which performs optical coherence tomography (OCT) utilizing an interference phenomenon of multi-wavelength light, is an apparatus capable of obtaining a tomographic image (tomogram) of a sample (e.g. a region of the fundus of the eye) with high resolution.

The OCT apparatus is becoming indispensable as an ophthalmological device for a retinal health specialist, for the purpose of viewing a selected part of a patient's retina.

The use of an OCT apparatus is not limited to ophthalmological applications, however, and is of use in other endothelial imaging such as in endoscopy or the surface imaging of skin or other organs.

US 2010/0014089 describes an OCT apparatus that creates a tomogram using interference light, the interference light arising from interference between measurement light from a sample arm of the OCT apparatus and reference light from a reference arm of the OCT apparatus. The sample arm reflects light from the examined object (the "sample") whereas the reference arm reflects light from a moveable reference mirror. The position of the reference mirror is set so that interference of light from the two arms gives an optimal tomogram. A photographing apparatus is provided which allows smooth adjustment of an optical path length of the measurement light to the examined object (such as an eye) in order to align the image of the object so that it has a desired position on the tomogram generated by the photographing apparatus. The initial position of the reference mirror is set to some midpoint in the movable range of the mirror, and is preferably set at a position in the reference arm of the OCT apparatus that is equivalent to a position in a sample arm of the OCT apparatus that is close to normal eye axial length.

US 7643154 B2 describes a conventional OCT apparatus that has a "changer" for changing a difference in optical path length between the sample arm optical path and the reference arm optical path. The conventional OCT apparatus description is incorporated herein by reference. The goal described in this document is to adjust a reference mirror in the reference arm optical path in order to adjust a position of a tomographic image in a "frame"; i.e. in the OCT image as viewed by a user. The way that this is done is by starting with the reference mirror at one end of its movable range, then checking where in the frame the tomographic image of the fundus of the eye is located. If there is no image in the frame, the reference mirror is moved along its movable range by set increments until the fundus tomographic image becomes visible. It may then be further moved until the fundus tomographic image is in a preferred position within the frame. The way that it is determined whether the fundus tomographic image is within the frame or not is by either a user looking at the frame (manual determination) or by pixel analysis of the frame (automatic determination).

US 7643154 B2 states that when a depth position in the eye corresponding to the position of the reference mirror exists within the vitreous body of the eye, the tomographic image of the fundus oculi does not show up within the frame. US 7643154 B2 does not take into account an artefact or condition in which a "frame" produced by the OCT apparatus has two modes: a first in which the tomographic image is correctly within the frame; and a second in which the "real" image of the fundus is outside of the frame, but a virtual, reflected (and therefore inverted) image of the fundus is present within the frame. Both the manual and automatic methods of determining whether the tomographic image of the fundus oculi is within the frame may give a false positive when the latter mode occurs. The two modes will be explained later. As the reflected image will have a different orientation from the real image, US 7643154 B2 does not address the problem of confirming both orientation and position of a tomographic image of a fundus oculi in an OCT frame.

US 7777893 B2 discloses an almost identical system to that of US 7643154 B2 (as does US 7982880 B2), but includes a noise value in its automatic evaluation of whether the tomographic image of the fundus oculi is present in the frame. Both US 7777893 B2 and US 7982880 B2 suffer from the same problems as US 7643154 B2.

Returning to US 2010/0014089, after calculating the image position within the tomogram or frame, a determination is made of whether the image orientation on the tomogram is normal or inverted by comparing dispersion correction data with a Fourier transform of the spectral intensity of the interference light. Two sets of dispersion correction data are calculated in advance. The first set of dispersion correction data is based on the tomogram having a normal orientation. The second set of dispersion correction data is based on the tomogram having a reflected or inverted orientation. As a consequence, a determination of whether the image orientation is normal or inverted can be made by comparing the observed spectral intensity with the two sets of dispersion correction data. Using this information, an adjustment is made to the optical path length, to align the object with the desired position on the tomogram in the correct orientation.

However, a disadvantage of the technique described in US 2010/0014089 is that two sets of dispersion correction data need to be calculated in advance, which requires time and resources, thereby placing a limitation on the provision of real-time imaging. Thus, there is a demand for an OCT apparatus that has an improved efficiency for aligning an object so that its image is in a desired position and orientation on a tomogram. A further disadvantage of the technique described in US 2010/0014089 is that it requires intentional hardware dispersion mismatch which limits effective imaging depth and sensitivity.

Further to the above, US 2010/0014089 describes a method for initialising the optical path lengths of the sample and reference arms. The initial position of the reference mirror is set somewhere in the middle of its movable range, with preferred position corresponding closely to the axial length for a typical eye.

However, a disadvantage of the technique described in US 2010/0014089 is that it is likely that the fundus image will not be present on a tomogram unless the eye has typical characteristics. Thus, there is a demand to provide an OCT apparatus that can efficiently accommodate a wide range of eye types, such as high myopic/hyperopic eyes.

US 2013/0182218 A1 first defines the term "coherence gate" as "a position at which [an] optical path length of the measuring light [of the OCT apparatus] becomes equal to the optical path length of the reference light". It goes on to say that "a tomographic image acquired in the [OCT] apparatus is acquired as an image corresponding to a distance from the coherence gate." The "coherence gate" is represented as a dotted line at the top of the "frame" in which the tomographic image of the fundus oculi is displayed. In US 2013/0182218 A1, the coherence gate has a starting position that is estimated as being within the eye, in front of the retina and is based on a minimum average length of an eye from the measurable front of the eye to the retina (20 - 30mm). The coherence gate is then brought closer to the retina step-by-step until the desired tomographic image can be acquired. If a signal is detected that indicates that the visible image of the tomographic image includes its reflected image, then it can be determined that the step-by-step movement of the coherence gate has gone too far and the coherence gate can be moved away from the fundus oculi position to obtain an optimum tomographic image position. The problem of how to establish if the starting point of the coherence gate means that only the mirror-image of the tomographic image is visible (i.e. knowing from the start in which mode the display is presented) in the frame is addressed only by the potentially erroneous method of using a starting position that is as likely as possible to be on a predetermined side (the inside) of the retina. Thus, determination of the orientation of the tomographic image is not considered. A problem with the method of US 2013/0182218 A1 is that in order to guarantee that the coherence gate is positioned within the eye, on the "correct" side of the retina, there is a risk that the initial position is very far from the retina and so the step-wise movement and measurement of the coherence gate position with respect to the retina may take a long time and have a high processing load.

A problem considered with respect to the examples cited above is how to view a selected part of a retina on a tomogram. This involves ensuring that the retina is in both a desired orientation and a desired position. It is desirable to improve the quality of real-time calculations so that corrections can be made to update an image if the object changes and also so that moving images can be viewed. Furthermore, it is beneficial to provide a tomogram that is suitable for viewing a broad range of eye types (e.g. myopic, etc.) and sizes.

Thus, there is a demand for a way to set the retinal image in the desired orientation and desired position in the tomographic frame that is fast and reliable and optimised for efficient and repeatable examination.

US 2011/0181889 describes an optical interference measuring method for which light emitted from a light source unit is split into measuring light and reference light, coherence light is detected that is obtained by interference of the reference light and the measuring light reflected from or scattered rearward from a measuring object, an optical-path length adjustment mechanism provided in the optical path of the reference light is driven to change the optical path length of the reference light, it is decided whether an image based on the detected coherent light is a normal image or a folded image based on the coherent light having varied with the change of the optical path length of the reference light, and the measuring object is measured from the detected coherent light based on a result of the decision about whether the image is a normal image or a folded image.

US 2009/0244547 describes an optical tomographic image system comprising an optical path length adjustor configured to set a first reference position of a measurement depth direction to an inner edge of a measurement range by adjusting an optical path length of a reference light, and an optical path length switching unit having a preset optical path length that provides a second reference position differing in measurement depth from the first reference position by a predetermined amount and configured to change the optical path length of the reference light or the optical path length of the reflected light adjusted by the optical path length adjustor so as to switch between the first reference positon and the second reference position. This system is capable of measuring a measurement region of interest at high resolution, regardless of the position of the measurement region of interest, in a swept source optical coherence tomographic imaging system employing a wavelength-swept light source.

**Summary of Invention**

Aspects of the present invention are set out by the independent claims.

**Brief description of the drawings**

FIGs. 1A and 1B are structural views of an OCT apparatus;
FIG. 2 is a block diagram illustrating a configuration of an image processing apparatus;
FIGs. 3A and 3B are explanatory views illustrating a 3D image-acquiring method;
FIG. 4A demonstrates how the tomogram is presented in the choroid mode; FIG. 4B demonstrates how the tomogram is presented in the vitreous mode; FIG. 4C demonstrates how the image-sensitivity of the tomogram is dependent upon the position of an image on the tomogram;
FIG. 5 is a flowchart of an overview of a method for viewing an object on a tomogram;
FIGs. 6A and 6B are flowcharts showing two possible techniques for initialising the view of the object on the tomogram;
FIG. 7 is a flowchart showing how the view of the object can be arranged in a desired position and a desired orientation;
FIG. 8 is a flowchart showing how the view of the object can be corrected;
FIG. 9 is a graph that shows a theoretical calculation of how the axial length of the eye is dependent upon a refraction error;
FIGs. 10A and 10B show examples of how the initial orientation could be determined by changing the position of the normal image by $-2*P_{RET}$; FIGs. 10C and 10D show examples of how the initial orientation could be determined by changing the position of the normal image by a combination of both $+P_{RET}$ and $+R_{DEST}$ in a single step;
FIGs. 11A, 11B, 11C and 11D show how the initial orientation could be determined by changing the position of the normal image by an amount $-R_{DEST}$;
FIGs. 12A, 12B, 12C and 12D show how the initial orientation could be determined by changing the position of the normal image by an amount $+R_{DEST}$;
FIGs. 13A, 13B, 13C and 13D show how the initial orientation could be determined by changing the position of the normal image by an amount $+P_{RET}$;
FIGs. 14A, 14B, 14C and 14D show how the initial orientation could be determined by changing the position of the normal image by an amount $-P_{RET}$;
FIG. 15A and 15B show examples of how the initial orientation could be determined by changing the position of the normal image by an arbitrary amount; and
FIG. 16 demonstrates an alternative technique for measuring the position of the object using the tomogram.

**Detailed description**

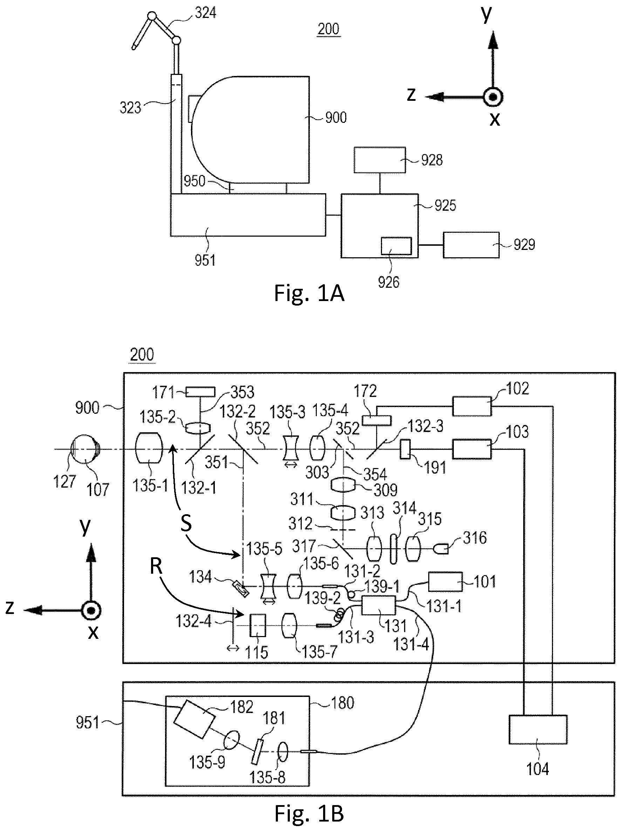

FIG. 1A is a side view of an ophthalmologic apparatus. An ophthalmologic apparatus (such as a fundus inspecting apparatus) 200 includes an optical head 900 that is a measuring optical system for taking an anterior ocular segment image and both a two-dimensional image and a tomogram of a fundus, and a stage portion 950 that is a movable portion capable of moving the optical head through the use of motors (not shown) in xyz-directions. A base portion 951 contains a spectrometer (described later). An OCT apparatus 200 may omit one or other of the anterior ocular segment imaging part and the two-dimensional fundus imaging part.

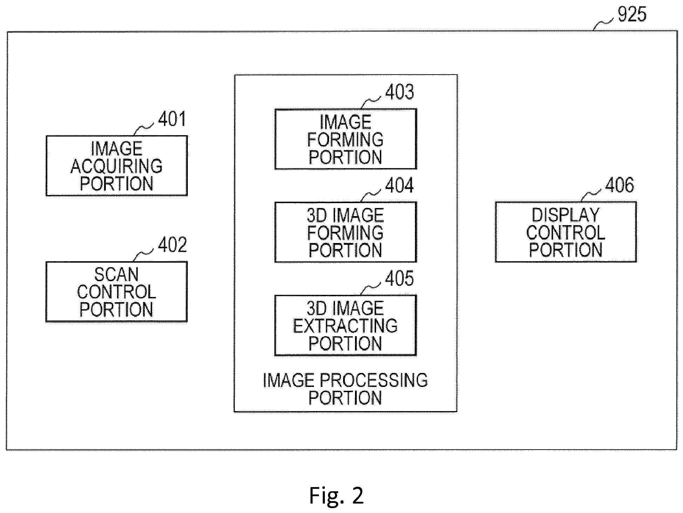

A computer 925 controls the stage portion 950 and generates a tomogram. Further details of the computer 925 are provided below with reference to FIG. 2.

A storage unit 926 also serves as a subject information storage portion and stores a program for taking a tomogram. An input portion 929 facilitates the provision of instructions to the computer 925. Specifically, the input portion 929 includes a keyboard and a mouse. A display 928 serves to present the tomogram, and facilitates the provision of instructions to the computer 925.

A jaw holder 323 holds a jaw and a forehead of a subject to urge the subject to fix an eye (eye to be examined) on light from an external fixation lamp 324.

Configurations of the measuring optical system and the spectrometer are described with reference to FIG. 1B.

First, an inside of the optical head 900 part is described. An objective lens 135-1 is set so as to be opposite an eye 107 to be inspected, and an optical path on an optical axis thereof is branched into an optical path 351 of an OCT optical system, an optical path 352 for fundus observation and fixation lamp, and an optical path 353 for anterior ocular segment observation depending on the wavelength band reflected or transmitted by a first dichroic mirror 132-1 and a second dichroic mirror 132-2. Out of lenses 135-3 and 135-4, the lens 135-3 is driven by a motor (not shown) for focus adjustment for a fixation lamp 191 and a charge-coupled device (CCD) 172 for fundus observation. The use of CCD 172 is optional, and instead it would be possible to use, for example, an Avalanche Photodiode.

Between the lens 135-4 and a third dichroic mirror 132-3, a perforated mirror 303 is arranged, and the optical path 352 is branched into the optical path 352 and an optical path 354.

The optical path 354 forms an illuminating optical system for illuminating the fundus of the eye 107 to be examined. On the optical path 354, there are arranged a light-emitting diode (LED) 316 serving as an illuminating light source for fundus observation that is used for positioning the eye 107, and a strobe tube 314 that is used for imaging the fundus of the eye 107. Condenser lenses 313 and 315 and a mirror 317 are further arranged on the optical path 354. Illuminating light emitted from the LED light source 316 and the strobe tube 314 is shaped into a ring-like light flux by a ring slit 312 and is reflected by the perforated mirror 303 so as to illuminate a retina 127 of the eye 107. Lenses 309 and 311 are further arranged on the optical path 354. The LED light source 316 has a central wavelength of approximately 780 nm, for example.

Beyond the perforated mirror 303 on the optical path 352 (as viewed from the eye), the optical path 352 is branched by the third dichroic mirror 132-3 into an optical path to the CCD 172 for fundus observation and an optical path to the fixation lamp 191 by using the extent of the wavelength band of the light.

The CCD 172 has a sensitivity corresponding to a central wavelength of the LED light source 316 that emits illumination light for fundus observation: for example, approximately 780 nm. The CCD 172 is connected to a CCD control portion 102. The fixation lamp 191 generates visible light to urge the subject to fix the eye. The fixation lamp 191 is connected to a fixation lamp control portion 103.

The CCD control portion 102 and the fixation lamp control portion 103 are connected to a computing portion 104, and data is input and output via the computing portion 104 to and from the computer 925.

A lens 135-2 and an infrared CCD 171 for anterior ocular segment observation are provided on the optical path 353 originally branched off the sample arm optical path by mirror 132-1. The CCD 171 has sensitivity corresponding to a wavelength of illumination light for anterior ocular segment observation (not shown): for example, approximately 970 nm. Further, an image-splitting prism (not shown) is arranged on the optical path 353. Thus, a distance in the z-direction of the optical head 900 part with respect to the eye 107 to be examined can be detected as a split image in the anterior ocular segment observation image. Use of the CCD 171 is referred to as use of the Anterior Preview modality.

The optical path 351 (branched off the sample arm optical path by mirror 132-2) forms the OCT optical system as described above and is used for taking a tomogram of the fundus of the eye 107 to be examined. An xy-scanner 134 scans light on the fundus. The xy-scanner 134 is illustrated as a single mirror, but performs scanning in 2-axis directions of x and y. Out of lenses 135-5 and 135-6, the OCT focus lens 135-5 is driven by a motor (not shown) for adjusting the focus of light emitted from a light source 101 through a fibre 131-2 connected to an optical coupler 131 onto the fundus of the eye 107. The focus adjustment causes the light from the fundus of the eye 107 to form an image in a spot shape to enter an end of the fibre 131-2.

Next, an optical path from the light source 101 to a reference optical system (reference arm R), and the spectrometer is described.

The arrangement includes the optical source 101, the optical coupler 131, a spectrometer 180, and the reference optical system R which includes a reference mirror 132-4 acting as an adjustable optical delay line, a dispersion compensation glass 115, mentioned above, an optical fibre 131-3 connected to the optical coupler 131 to be integrated, and a lens 135-7.

The ophthalmologic apparatus 200 includes a sample arm S, which is formed by the optical path from the eye 107 through the objective lens 135-1 and the optical path 351 of the OCT optical system up to the optical coupler 131.

The above-mentioned components constitute a Michelson interferometer. The light emitted from the light source 101 is split into measuring light on the optical fibre 131-2 side and reference light on an optical fibre 131-3 side through the optical fibre 131-1 via the optical coupler 131.

As an alternative to the Michelson interferometer, a Mach-Zehnder interferometer may instead be used. A Mach-Zehnder interferometer may be used in the case where an optical amount difference between the measuring light and the reference light is large, and a Michelson interferometer may be used in the case where the optical amount difference is relatively small.

The measuring light illuminates the fundus of the eye 107 to be observed through the optical path of the OCT optical system described above and reaches the optical coupler 131 through the same optical path as a result of the illumination light reflecting and scattering from the retina 127.

The reference light reaches the reference mirror 132-4 through the optical fibre 131-3, the lens 135-7, and the dispersion compensation glass 115 inserted for matching the dispersion of the measuring light with that of the reference light, and is reflected from the reference mirror 132-4. Then, the reference light returns through the same optical path and reaches the optical coupler 131.

The optical coupler 131 combines the measuring light with the reference light to form interference light. In this case, interference occurs when an optical path length of the measuring light and an optical path length of the reference light become substantially equal to each other.

The range for which the reference light and the measuring light are coherent is referred to as the zero delay line (ZDL), as will be described later. Modifying the range for which the reference light and the measuring light are coherent is performed by changing the optical path length of the reference light or the optical path length of the measuring light, which moves this ZDL allows the relative position of the retinal image and the ZDL on the tomogram to be controlled. A relative movement between the ZDL and the retinal image is perceived by a user to be a movement of the retinal image because the position of the ZDL with respect to the tomogram frame is kept constant.

The reference mirror 132-4 is adjustable in an optical axis direction (shown by a double-headed arrow) by a motor and a drive mechanism (not shown) and is capable of adjusting the optical path length of the reference light so that it matches that of the measuring light, the optical path of the measuring light varying depending on the characteristics of the eye 107 to be examined. The interference light is guided to the spectrometer 180 through the optical fibre 131-4.

An eye position relative to an OCT optical path determines where on the tomogram the retinal image will appear. If the eye is too far away, the retinal image drops off the bottom of the image. If the eye is too close but within a certain range, the image of the retina will be inverted. An optimum or most sensitive distance will be defined by the relative lengths of the sample optical pathway and the reference optical pathway of the OCT apparatus 200.

Further, a polarization adjusting portion 139-1 is provided for the measuring light in the optical fibre 131-2. A polarization adjusting portion 139-2 is provided for the reference light in the optical fibre 131-3. Those polarization adjusting portions each have a part in which the optical fibre is looped several times. This looped part is rotated about the longitudinal direction of the fibre to twist the fibre. In this manner, the polarization state of each of the measuring light and the reference light can be adjusted and matched to each other. In this apparatus, the polarization state of each of the measuring light and the reference light is adjusted in advance and fixed.

The spectrometer 180 includes lenses 135-8 and 135-9, a diffraction grating 181, and a line sensor 182.

The interference light emitted from the optical fibre 131-4 is substantially collimated through the lens 135-8 and dispersed by the diffraction grating 181 to form an image on the line sensor 182 by the lens 135-9.

Next, the periphery of the light source 101 is described. The light source 101 is a super luminescent diode (SLD) that is a typical low-coherent light source. Light emitted from the light source 101 has a central wavelength of 855 nm and a wavelength band width of about 100 nm. In this case, the band width influences a resolution in an optical axis direction of a tomogram to be acquired, and hence, is an important parameter. Further, although the SLD is selected, the type of the light source 101 is not particularly limited as long as the light source 101 is capable of emitting low-coherent light, and amplified spontaneous emission (ASE) or the like may also be used.

Considering the measurement of an eye, near-infrared light is suitable for the central wavelength. Further, it is desired for the central wavelength to be a shortest possible wavelength because the central wavelength influences a resolution in a lateral direction of a tomogram to be acquired. The central wavelength is set to 855 nm, as an example.

FIG. 2 is a schematic view illustrating the function of the computer 925. A method of creating a tomogram using the fundus inspecting apparatus 200 is described below with reference to the functional block diagram illustrated in FIG. 2.

The computer 925 (specifically, a processor included in the computer 925) executes, for example, a program stored in the storage unit 926 (see FIG. 1A) to function as an image-acquiring portion 401, a scan control portion 402, an image-forming portion 403, a 3D-image-forming portion 404, a 3D-image-extracting portion 405, and a display control portion 406.

The fundus inspecting apparatus 200 controls the xy-scanner 134 to generate a tomogram of a desired part of the fundus of the eye 107. Specifically, the scan control portion 402 controls the xy-scanner 134.

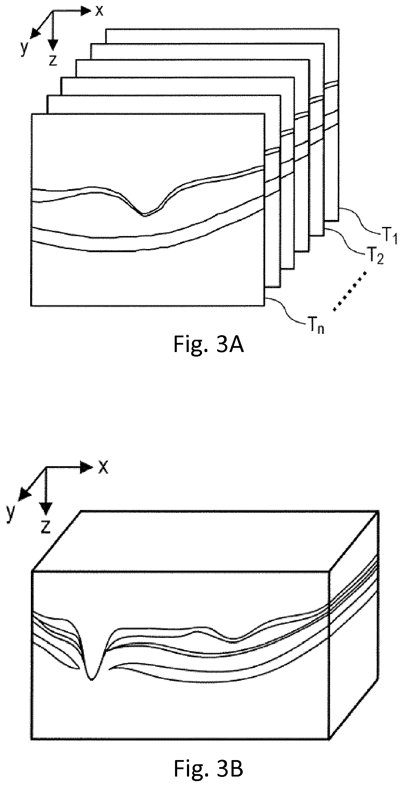

An image-processing method using the fundus-inspecting apparatus 200 will now be described. When a scan pattern is selected via the input portion 929, regardless of the selected scan pattern, the scan control portion 402 controls the xy-scanner 134 to perform raster scanning. Then, the image acquiring portion 401 acquires a tomogram based on a signal received by the line sensor 182. That is, the image-acquiring portion 401 serving as an acquiring unit for acquiring a tomogram group acquires images for forming a 3D tomogram. The xy-scanner 134 scans the measuring light in an x-direction of FIG. 3A, and the line sensor 182 images information a predetermined number of times from an imaging range in the fundus in the x-direction.

A Fourier transform (FT) or alternative image reconstruction method such as FFT (fast FT), DFT (discrete FT), NUDFT (non-uniform DFT) or TV regularisation is performed on a luminance distribution obtained on the line sensor 182 at a certain position in the x-direction, and a linear luminance distribution (in the z-direction as shown in FIG. 3A) obtained by the FT is converted into density or colour information. This converted information is referred to as an A-scan image. A plurality of A-scan images are taken to give one B-scan image $T_1$ in the xz-plane of FIG. 3A. The scan position in the y-direction as shown in FIG. 3A is moved, and the scanning in the x-direction is performed again so that a plurality of B-scan images are acquired. That is, a plurality of tomograms $T_1$ to $T_n$ that are acquired at different positions on the eye to be examined and extend in parallel to each other are acquired as a tomogram group. Based on the images acquired by the image acquiring portion 401, the 3D image forming portion 404 forms a 3D image illustrated in FIG. 3B. Here, n tomograms suitable for 3D image formation are obtained, wherein the value of n may be increased or decreased depending on the resolution of the image to be extracted.

In summary, the OCT apparatus 200 is capable of splitting light having low coherence into reference light and measuring light, and irradiating an object to be examined with the measuring light. After the measuring light has illuminated the object, the measuring light interferes with the reference light, to give information regarding the depth-direction of the object and thereby to measure one or more layers of the object. Further, the OCT apparatus 200 can obtain a tomogram with high resolution by scanning the measuring light over the sample. Using this technique, the tomogram of the fundus of the eye is acquired to be widely used for ophthalmologic diagnosis of the retina and the like.

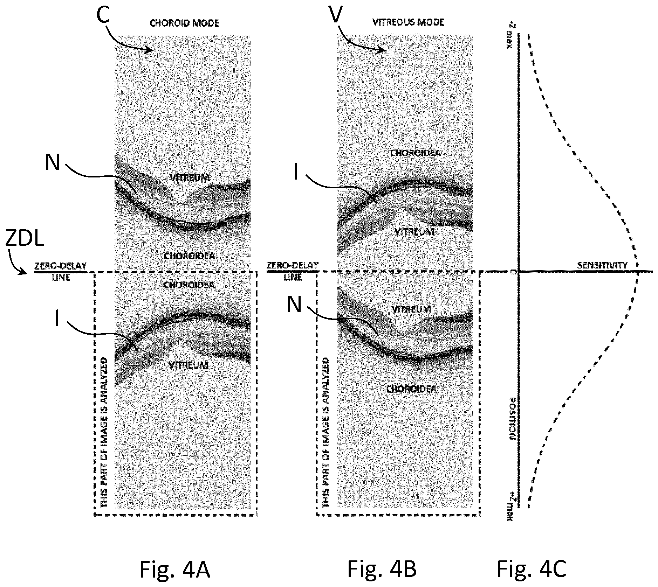

FIGs. 4A, 4B and 4C demonstrate how a retinal image can be viewed on a tomogram obtained using OCT. The orientation of the retina 127 on the tomogram can be presented in either a choroid mode C (FIG. 4A) or a vitreous mode V (FIG. 4B). The dashed boxes in FIGs. 4A and 4B indicate the part of the tomogram that is typically presented to a user on the display 928.

The graph (FIG. 4C) shows how the sensitivity of the OCT apparatus 200 is dependent upon the distance of the object from the zero delay line (ZDL). The ZDL (or coherence gate as it is called in US 2013/0182218) corresponds to the condition of the interferometer being such that the optical path length difference between the reference arm R and the sample arm S is equal to zero. The term "optical path length difference" refers to the optical delay between the reference arm R and the sample arm S which could be induced by geometrical path length difference (e.g. by moving a reference mirror 132-4) or by optical path length variation (see for example Polish patent PL 218325B1) or providing a phase delay to the reference light (e.g. by grating-based phase control optical delay line as described in US6111645A) or by any other method.

The z-axis is directed vertically downwards in FIGs. 4A, 4B and 4C. The sensitivity of the OCT apparatus 200 is greatest in the vicinity of the ZDL. The sensitivity decreases for positions that are further away from the ZDL along the z-axis. This decrease in sensitivity is an inherent disadvantage of any spectrometer-based OCT system. When viewed on the display 928, the ZDL is most usually at the top of the display area even though the relative position of the ZDL with respect to the retinal image may change. Thus, it is the retinal image that will appear to move in the frame. Accordingly, the user will be able to analyse data that has a positive value along the z-axis, arranged so that the ZDL, for which z = 0, is at the top of the tomogram.

In the choroid mode C, the retinal image is oriented so that the choroidea of the eye (the area behind the retina) is closest to the ZDL. In the vitreous mode V, the retinal image is oriented so that the vitreum of the eye (the area within the retina) is closest to the ZDL. Accordingly, if the user wants to examine the choroidea in greater detail, then the choroid mode C will provide the highest sensitivity. Similarly, if the user wants to examine the vitreum in greater detail, then the vitreous mode V will provide the highest sensitivity. The way to do this is to have the ZDL as near as possible to the retina 127, but on either the choroidal or vitreal side of the retina 127. In order to view the desired mode, advantage is taken of the reflection of the image in the tomogram frame when the ZDL passes through the retina 127.

A typical OCT device acquires real signals, which are subsequently analysed by Fourier transform (FT). It is well known that FT of a real signal generates a peak at the positive side of the FT domain, and its counterpart on the negative side. Thus, FT of real signal cannot be used to recognize whether the path length difference is positive or negative. Keeping this in mind, in case of retinal imaging, we distinguish two imaging modes. In the first mode, the retina 127 is located with respect to the reference mirror 132-4 to provide a positive path length difference (i.e. the optical path length of the sample arm S is longer than the optical path length of the reference arm R). The view of the retina 127 in the first mode is called the vitreous mode V and is referred to as the normal image N of the retina 127, and the visible part of the tomogram has the vitreum nearer the zero delay line. In the second mode, the retina 127 is located with respect to the reference mirror 132-4 to provide a negative path length difference (i.e. the optical path length of the sample arm S is shorter than the optical path length of the reference arm R). The view of the retina 127 in the second mode is called the choroid mode C and is referred to as the inverted image I of the retina 127, because the choroidea is located nearer the zero delay line than the vitreum. The inverted image I is an inverted version of the normal image N.

A user is often motivated to view a chosen part of the retina 127, depending on the patient's condition that is subject to examination. The tomogram can be improved by modifying the image so that the chosen part of the retina 127 is viewed in the vicinity of the ZDL where the OCT apparatus 200 provides its highest sensitivity.

The following describes how a position and orientation (based on mode) of the retinal image in the tomogram frame may be determined and adjusted. In summary, the following steps are taken and the steps relevant to the positioning of the image of the retina 127 on the tomogram will be described hereinafter:

1. Turn on system including Anterior Preview, SLO, OCT and pre-determine the desired position of the retinal image in the tomogram $R_{DEST}$;
2. Set reference mirror 132-4 to starting position;
3. Run Anterior Preview modality (135-2, 171) with split-prism range finder to determine position of anterior segment of the eye.
4. Find distance $\Delta Z$ of eye position against Working Distance WD by analysing the split-prism image.
5. Find a refraction error F of the eye using an SLO image and the focus lens 135-3;

**6.** Set OCT focus lens 135-5 to the found value F;

**7.** Estimate eye ball length D(F) based on a model eye;

**8.** Calculate a position $CG_{PROG}$ of the zero delay line (or coherence gate as it is called in US 2013/0182218) (e.g. by calculating the optical path length or the position of the reference mirror 132-4) to obtain an OCT image;

**9.** Adjust the zero delay line (optical path length or reference mirror 132-4) to the calculated position $CG_{PROG}$;

**10.** Make a test move to confirm orientation of the signal; and

**11.** Make fine correction of image position to bring the image to desired or destination position $R_{DEST}$.

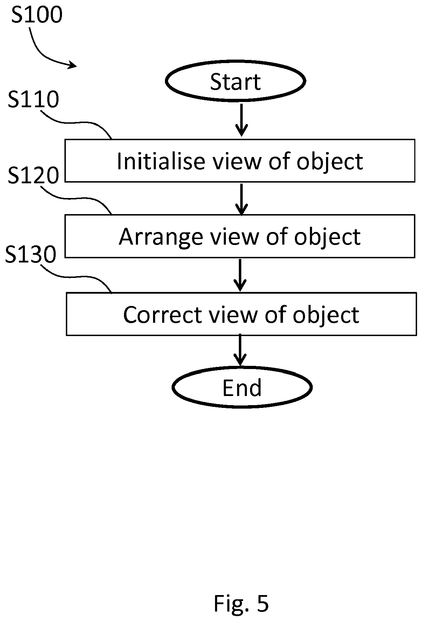

FIG. 5 provides an overview of a method S100 for achieving the desired orientation and the desired position.

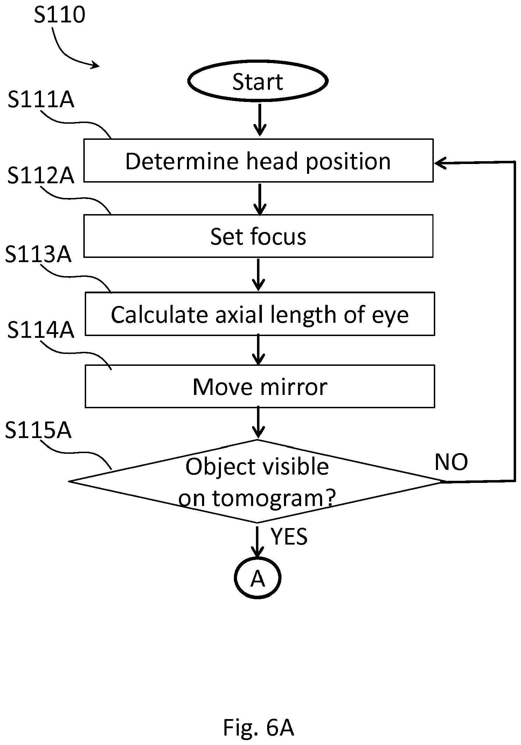

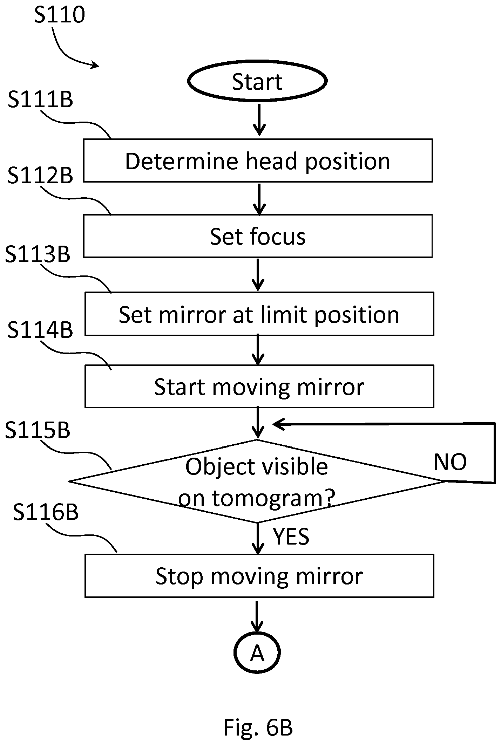

In step S110, the view of the retina 127 is initialised, which means that the OCT apparatus 200 is arranged so that the retina 127 is in an initial position so that its image can be viewed on the tomogram. This step may require no more than switching the illumination light on because it is possible that the retina 127 could already be aligned and thus its image viewable on the tomogram without any further action needed to initialise the image. Further detail of this step is provided below with reference to FIGs. 6A and 6B. On the other hand, some manipulation of the eye position, reference mirror position or relative position between the OCT apparatus 200 and the eye may need to be performed for the initial view of the retina 127 to be acceptable.

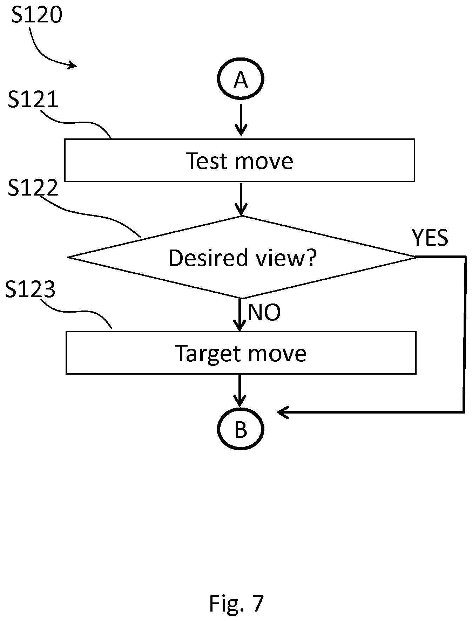

In step S120, the image of the retina 127 is arranged so that the retina 127 is viewed at the desired position and in the desired orientation, so that the chosen part of the retina 127 that is to be viewed is positioned closest to the ZDL (z = 0 in FIGs. 4A, 4B and 4C). Further detail of this step is provided below with reference to FIG. 7.

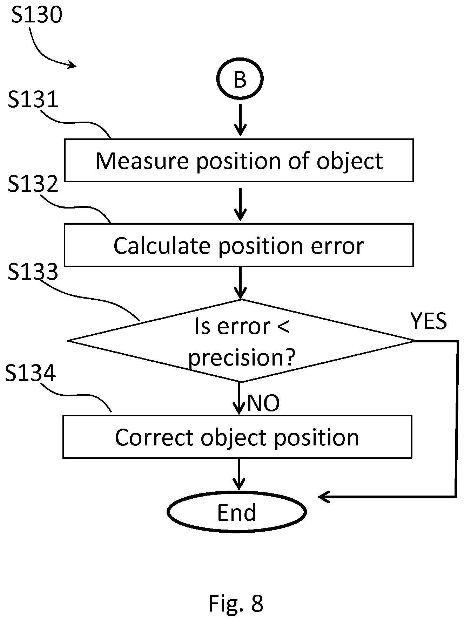

In step S130, the image position is corrected. This takes into account errors that could have occurred, for example, because of movement of the retina 127 relative to the OCT apparatus 200 between A-scans or B-scans. An advantage of correcting the errors is that movement during the previous steps S110 and S120 can be corrected. Another advantage of correcting the errors is that images can be updated in real-time. This step is not essential if the retina 127 has not moved or if the correct image of the retina 127 is already displayed and does not need to be corrected after step S120. Also, this step is not essential if the user does not require an update to the image in real-time or if the user is willing to use the images without having the errors corrected. Further detail of this step is provided below with reference to FIG. 8.

FIGs. 6A and 6B show two possible methods for configuring the OCT apparatus 200 to arrange the initial view the retina 127 on the tomogram according to the initialisation step S110. The initial positions of the retina 127 and of the mirror 132-4 define the initial optical path lengths.

FIG. 6A provides a method which involves estimating a value of the position of the eye and setting the initial optical path length of the reference arm R by moving the reference mirror 132-4 directly to the estimated position. The OCT apparatus 200 is then arranged to continue searching for a view of the retina 127 by moving the reference mirror 132-4 in either direction until the retina 127 has been detected.

FIG. 6B provides a method which involves positioning the mirror 132-4 at a limit position of its movement range and modifying the optical path length of the reference arm R by moving the reference mirror 132-4 in one direction until a view of the retinal image is displayed on the tomogram.

The retinal image on the OCT has the highest intensity when the beam is focussed on the retina 127. Manipulating the OCT focus is decoupled from the manipulation of the zero delay line. As a consequence, setting the focus of the beam on the retina 127 in advance allows the image to be subsequently optimised by tuning the zero delay line.

The implementation of the method shown in FIG. 6A comprises the following steps:

In step S111A, the head position of the subject is determined, which provides information on the distance (working distance Z) between the anterior ocular segment (i.e. the cornea of the eye) and the input lens 135-1 of the ophthalmic apparatus. A movement of the eye 107 being examined ($\Delta Z$) is determined relative to this working distance Z. A prism-split method is used to determine $\Delta Z$. When the eye is at the working distance Z, the $\Delta Z$ becomes zero and the image is not split by prisms. When the eye is out of working distance Z, the image is split by prisms and this can be processed to find actual $\Delta Z$ value which corresponds to how far from the working distance Z that the eye is.

In step S112A, the fundus viewing system is used to set the focus of the fundus to the estimated position. The view of the fundus is set either automatically or manually. Setting the focus on the fundus is performed by estimating a distance from the measurable anterior segment of the eye to the fundus. There are several ways in which this can be accomplished, as described below.

In step S113A, a calculation is made of the axial length of the eye (D). The value of D could be an estimate or a determination based upon known information relating to characteristics of the eye. For example, the axial length could be determined as a function of a refraction error (F) of the eye (typically determined using another modality such as SLO), and which will allow the calculation of D(F) using known algorithms, for example by using Zemax™ Optics software. One of the methods that can be used to determine F based on an SLO image is described in EP 2702933 A1. Alternatively, the axial length could be estimated based on a library of prior eye ball length measurements. However, the axial length D being determined using the refraction error F is described in more detail below. In step S114A, the reference mirror 132-4 is moved to a position $CG_{PROG}$ that is calculated to provide a view of the retina 127. Thus $CG_{PROG}$ is the initial position of the zero delay line, for which the retina 127 is visible on the tomogram. The OCT focus is set after determination of the length of the eye, although it is advantageous to set the OCT focus before the step in which the current position of the retina ($P_{RET}$) is determined. The calculation of $CG_{PROG}$ is determined using:

$$CG_{PROG} = D + \Delta Z +/- R_{DEST}, \qquad \text{(Equation 1)}$$

where:

$R_{DEST}$ is the destination position of the retinal image on the tomogram;
D is the axial length of the eye; and
$\Delta Z$ is the movement distance of the eye as above. Thus, this equation links the actual position of the retina 127 in the subject (via the $CG_{PROG}$) and the destination position of its image on the tomogram.

The position of the retina 127 on the tomogram can be determined by using the following method. At first, the set of A-scans is selected. In particular, all A-scans can be taken into account. In the next step, the noise on selected A-scans is cut by using a binary threshold. Next, the position of the retina 127 on each A-scan is calculated as the geometric center of pixels with value larger than 0 making up the A-scan. The position of the retina 127 on the tomogram is an averaged value of the geometric center calculated for the A-scans. The position is expressed in units of pixels. In order to convert the position to micrometers the position in pixels is multiplied by a certain scaling factor. The scaling factor corresponds to the size of a single pixel of a tomogram in the A-scan direction. The scaling factor can be also determined experimentally by using a mirror as an object and performing two tomogram images corresponding to two different positions of the mirror. Knowing the two positions of the mirror and finding a location of the surface image on the tomogram, one can find the scaling factor.

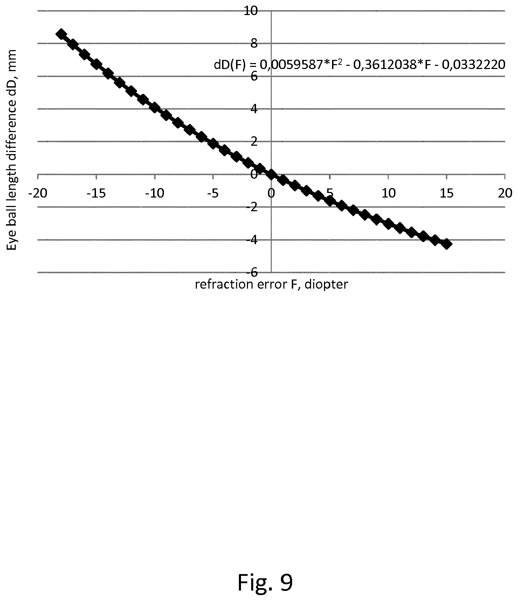

FIG. 9 shows the relative change of eye ball length dD as a function of the refractive error F. Thus, the eye ball length D(F) can be estimated as $D(F) = D_0 + dD(F)$, where $D_0$ is an eye-ball length of a normal eye. The initial position can be estimated for any eye with a certain refraction error F using equation 1.

In principle, when D is very accurately determined, it is possible to set the retina 127 in the desired orientation and desired position in one step using equation 1. Unfortunately, a high accuracy of D requires a very accurate model of the eye being measured. As a consequence, setting the retinal image in the desired orientation and the desired position is likely to require more steps in addition to step S110.

A possible technique would be to move the reference mirror 132-4 to obtain the desired orientation in one step. However, it has been discovered by the inventors that, in order to obtain a desired orientation, fewer moves of the reference mirror 132-4 are typically required if the initial orientation has the opposite mode from the desired orientation (i.e. initialising the vitreous mode V if the choroid mode C is desired; or initialising the choroid mode C if the vitreous mode V is desired). This will be explained below with reference to a parameter $\alpha$ used to determine the movement of the zero delay line. Advantageously, using fewer moves reduces the setting-up time.

The sign before $R_{DEST}$ in equation 1 is "-" if the vitreous mode V is to be set and "+" if the choroid mode C is to be set. The reason for this is that $R_{DEST}$ is an absolute distance from the ZDL. If the ZDL is to be in front of the retina 127 so that the vitreous mode is achieved, the zero delay line position must be shorter (closer) than the actual length to the retina 127 by $R_{DEST}$. For the choroid mode C, the ZDL position must be longer (farther) than the actual length to the retina 127 so that it is behind the retina 127 and so the $CG_{PROG}$ position of the ZDL adds $R_{DEST}$ to the length of the eye.

Accordingly, by moving the reference mirror 132-4 to the calculated position, it is intended that the retinal image will be visible on the tomogram.

In step S115A, it is determined whether the retina 127 is indeed visible on the tomogram. If the retina 127 is not visible, (NO in step S115A), this indicates that a failure has occurred (e.g. the axial length of the eye has been mis-estimated), and so the method returns to step S111A so that these steps can be repeated. However, if the retina 127 is visible (YES in step S115A), step S110 ends and the method continues to step S120.

In FIG. 6B, an alternative method is provided for positioning the retinal image on the tomogram.

Step S111B corresponds to S111A. However, this step is optional. It is not necessary to determine the head position if instead the user waits for the image to appear on the tomogram.

Step S112B corresponds to S112A.

Step S113B involves setting the reference mirror 132-4 at a limit position of its moving range (e.g. to make the reference arm R optical length a maximum or minimum length available with just movement of the reference mirror 132-4). The reference mirror 132-4 is then moved across its available range until an image of the retina 127 is visible on the tomogram (S114B to S116B). Accordingly S115B differs from S115A because as an alternative to repeating the previous steps again, instead the tomogram is monitored until the retina 127 becomes visible.

The above methods are distinguished from US 2010/0014089, for which the mirror is set at the middle of its moving range and must be moved back and forth iteratively until the correct position is found. Advantageously, the present methods (FIGs. 6A and 6B) ensure that the image of the retina 127 is viewable from a broader range of eye types that do not have typical characteristics.

Consequently, as shown in FIGs. 6A and 6B, step S110 ends when an initial view of the retina 127 can be observed on the tomogram (indicated in the drawings by the label A). At this stage, the orientation of the retina 127 could be either a normal image N of the retina 127 in the choroid mode C (FIG. 4A) or an inverted image I of the retina 127 in the vitreous mode V (FIG. 4B).

The normal image N has a corresponding inverted image I, each of which look like mirror images of one another. When a normal image N crosses the ZDL, it overlaps the corresponding inverted image I. It is because of the mirror image that the choroid mode C exists, because the user is actually viewing the inverted image I of the retina 127. In practice, when a retina 127 is observed, there are a range of values of $\Delta r$ which correspond to the different layers of the retina 127. Thus, when the range of values of $\Delta r$ overlap with the ZDL, their counterparts also overlap with the ZDL, which causes the normal image N and the inverted image I to overlap one another. This overlap causes a distinct change in intensity of the image, which can be used to recognise an overlap automatically by a processing apparatus. The distinct change in intensity is an indication that the ZDL is overlapping with the retina 127 and that the mode of the tomogram is changing.

The user does not necessarily know which orientation they are seeing in their visible tomogram (outlined with the dashed boxes in FIGs. 4A and 4B), and therefore whether they are seeing the normal N or inverted image I of the retina 127. Similarly, a processing apparatus will not necessarily recognise the mode from analysing the pixel intensity of the image. The next steps have the intention of moving the retinal image on the tomogram to the correct position and orientation despite this lack of knowledge by the user or processing apparatus.

FIG. 7 shows a method S120 for moving the view of the retina 127 from the initial view (label A) to the desired view (label B).

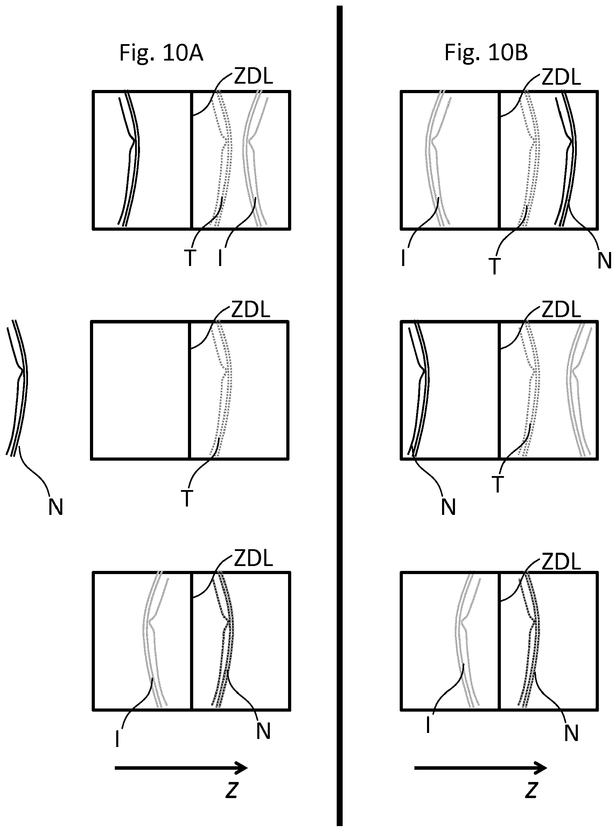

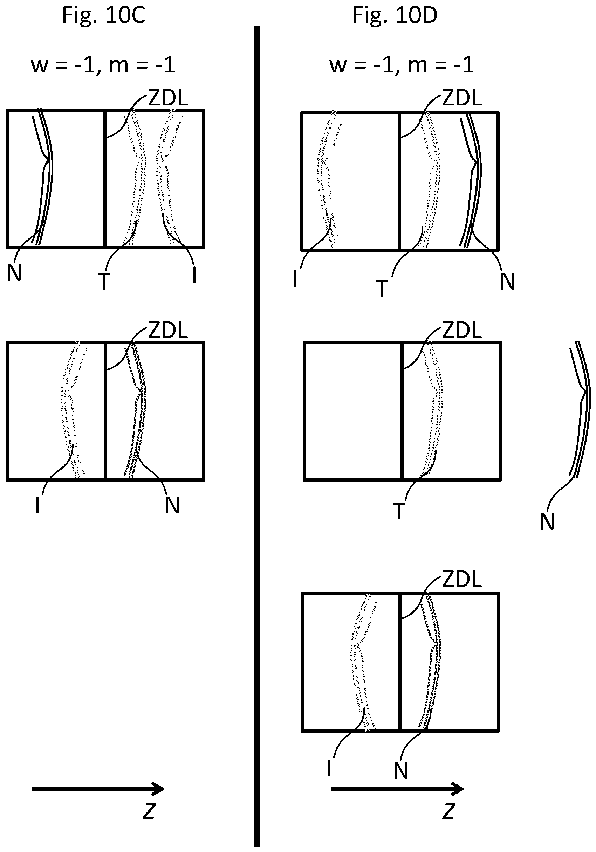

In step S121, a test move of the reference mirror 132-4 is made, so that the user can establish whether the tomogram is displaying a normal or an inverted image I. Examples of how the test move is done in order to determine the initial orientation will be described shortly, with reference to FIG. 10. The test move thereby allows the initial orientation of the image to be established, and using this information the desired orientation can be obtained. Advantageously, this method simplifies the determination of the initial orientation, because there is no need for the user to identify the initial orientation of the image with some other complicated method such as pixel-by-pixel comparisons with standard images. The omission of requiring the user to identify the initial orientation by instead performing the test move enhances the speed and efficiency of the OCT apparatus 200. Options are provided below for selecting the test move S121 that is to be made.

In step S122, a measurement is performed, which is used to determine whether the test move caused the image to move to the desired position and the desired orientation or not. By determining whether the image in the tomogram moved in the desired direction, valuable information is gained about the original orientation that resulted from the initialisation step S110. Consequently, the measurement S122 allows the initial orientation to be inferred. When the initial orientation is known, a final target orientation and position may more easily be calculated.

If the outcome of the determination S122 of whether the desired view has been achieved indicates that the tomogram is in the desired orientation and desired position (YES in step S122), then no further arrangement of the OCT apparatus 200 is required. Thus, process S120 ends. An advantage of establishing that the desired view was achieved when performing the test move (YES in step S122) is that the target move is neither calculated nor performed, and so processing is more efficient because fewer steps are required.

However, if the outcome of the determination S122 of whether the desired view has been achieved indicates that the image of the retina 127 is not in the desired orientation and desired position (NO in step S122), then further arrangement of the OCT apparatus 200 is required. An advantage of establishing that the desired view was not achieved when performing the test move is that valuable information is obtained that facilitates the calculation of how to achieve the desired view in step S123, as will be explained below.

In step S123, the initial view resulting from step S110 and the measured information regarding whether a test move gives rise to a desired target position for the image in step S122 are used to calculate a target move that will allow the desired view to be presented on the tomogram. After calculating the target move that will obtain the desired view (which will be explained below), the target move is performed. Process S120 ends once the desired orientation is displayed on the tomogram by the OCT apparatus 200 (indicated in the drawings by label B).

FIG. 10 provides examples of how to arrange the view of an object according to step S120. These examples demonstrate ways in which the test move (step S121) can be chosen. The test move S121 involves moving the image of the retina 127 obtained during initialisation S110 by a predetermined amount in a predetermined direction in order to determine the orientation of that image that was in fact obtained during the initialisation S110.

The user is motivated to move a portion of the image of the retina 127 towards the ZDL. However, after the initialisation step S110, the user does not know whether the OCT apparatus 200 is in the choroid mode C (FIG. 4A) or the vitreous mode V (FIG. 4B). Because the user does not know the initial orientation, this means that the user does not know the direction in which the image of the retina 127 will move when the user moves the reference mirror 132-4 in order to change the position of the retina 127 on the tomogram. In other words, the user may move the reference mirror 132-4 of the OCT apparatus 200 in a first direction, thinking they are moving the retinal image toward the ZDL only to find that they have been looking at the inverted image I and the image moves away. A test move is used to establish whether it is the normal image N or inverted image I that is in the screen viewable by the user. The test move does this by moving the reference mirror 132-4 in a predetermined direction by a predetermined amount. If a normal image N was viewed, the image will behave in a first way whereas an inverted image I will behave in a second way.

FIG. 10 indicates the relative positions of the normal image N, the inverted image I, and the desired position T. For each imaging window shown, the right hand side is visible to the user. The direction of the z-axis is towards the right of the drawing.

FIG. 10 provides examples of the display of the retinal image on the tomogram. For each of the examples provided in FIG. 10, the top drawing shows the configuration after the initialisation of the image S110. The middle drawing shows the configuration after performing the test move S121. The bottom drawing shows the configuration after completion of the process S120, except in the case where no target move is required (as shown by FIG. 10D).

For the examples shown in FIG. 10, the desired view is the vitreous mode, with the vitreum closer to the ZDL that is in the centre of each imaging window and to the right of the ZDL so that it is visible to the user. FIGs. 10A and 10B show a test move for which the normal image N is moved to the left (-z direction). FIGs. 10C and 10D show a test move for which the normal image N is moved to the right (+z direction). In FIGs. 10A and 10C, the initial view is of the inverted image I, whereas in FIGs. 10B and 10D, the initial view is of the normal image N.

For the first example shown in FIG. 10A, a view of the retina 127 is visible although the user does not know that the initial view from step S110 corresponds to an inverted image I. Moving the normal image N to the left (test move S121) sends it off the side of the image, and consequently no image is viewable after the test move S121. The target move S123 can then be performed in the opposite direction plus a distance appropriate to bring the image to the desired position $R_{DEST}$. In other words, moving the image in the -z direction by a known amount has caused it to disappear from the tomogram frame so it can be deduced that it was the inverted image that was visible. The next step is to move the image in the +z direction by the same known amount to get it back to where it was initially then to add a further amount to get it to desired position $R_{DEST}$.

For the second example shown in FIG. 10B, a view of the retina 127 is visible although the user does not know that the initial view from step S110 corresponds to a normal image N. After the test move S121, the normal image N has been moved to the left, so that the inverted image I becomes visible. The target move S123 can then be performed in the opposite direction plus a distance appropriate to bring the image to the desired position $R_{DEST}$.

For the third example that is shown in FIG. 10C, a view of the retina 127 is visible although the user does not know that the initial view from step S110 corresponds to an inverted image I. Moving the normal image N to the right by a predetermined distance during the test move S121 therefore means that the normal image N moves into the visible zone on the right hand side and, in this case, into the desired position $R_{DEST}$ thanks to the predetermined distance being appropriate. In this example, it is not necessary to perform the target move, because the assessment of whether the desired view had been achieved (YES in step S122) indicates that the target move S123 does not need to be performed.

For the fourth example that is shown in FIG. 10D, it is the normal image N that is visible in the right-hand side and so moving it to the right sends it off the side of the image so that no image is viewable after the test move S121. The target move S123 can then be performed in the opposite direction plus a distance appropriate to bring the image to the desired position $R_{DEST}$.

For the examples shown in FIG. 10, the target image T was in the vitreous mode V. Analogously, similar examples can be given where the target image T is in the choroid mode C.

After the test move S121, the user will be able to identify the initial orientation that had been obtained previously for the initialisation position S110. In other words, a prediction is made about the orientation of the image of the retina 127, and then this prediction is tested in order to arrange the image in the desired orientation. Advantageously, by predicting the orientation and testing the prediction, only one prediction is required. If the predicted orientation is determined to be incorrect, then the correct orientation can be inferred by process of elimination to be the opposite orientation to the predicted orientation.

Possible test moves include:

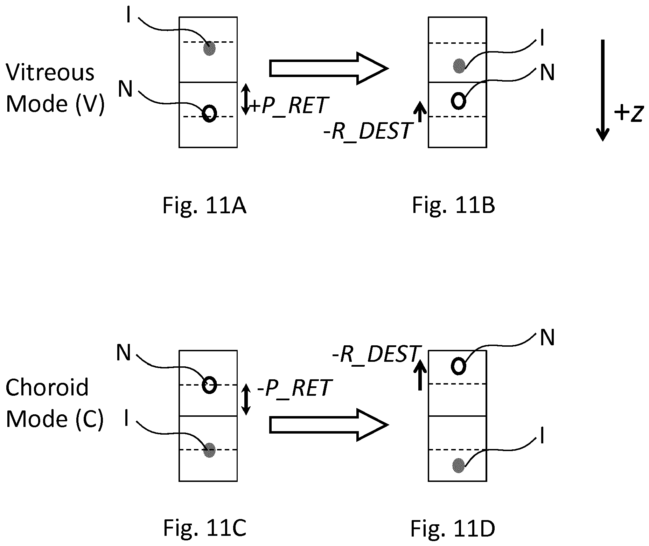

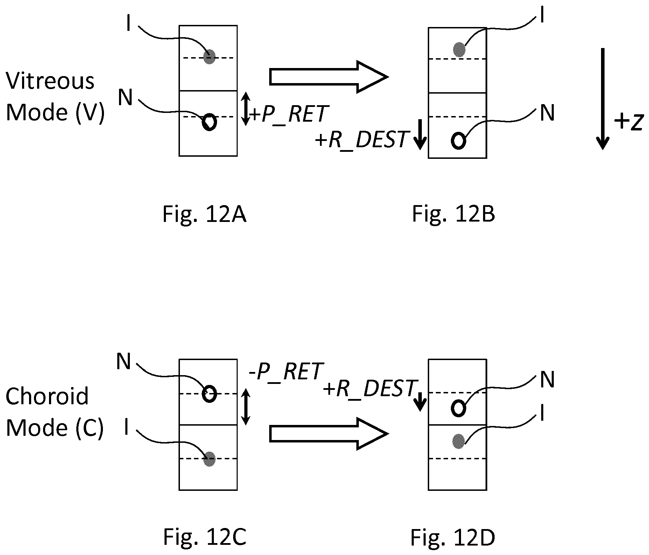

Option 1: Testing whether the predicted orientation is correct by moving the initial view of the retina 127 by the amount $+/-R_{DEST}$ (the desired distance from the ZDL to which the user wants to move the image of the retina 127) in a predetermined direction (+/-z). FIGs. 11 provide a schematic for a test move of $-R_{DEST}$, whereas FIGs. 12 provide a schematic for a test move of $+R_{DEST}$.

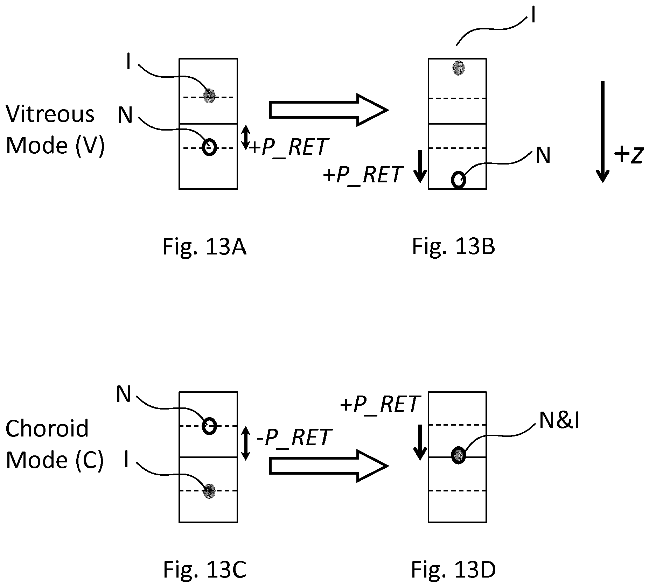

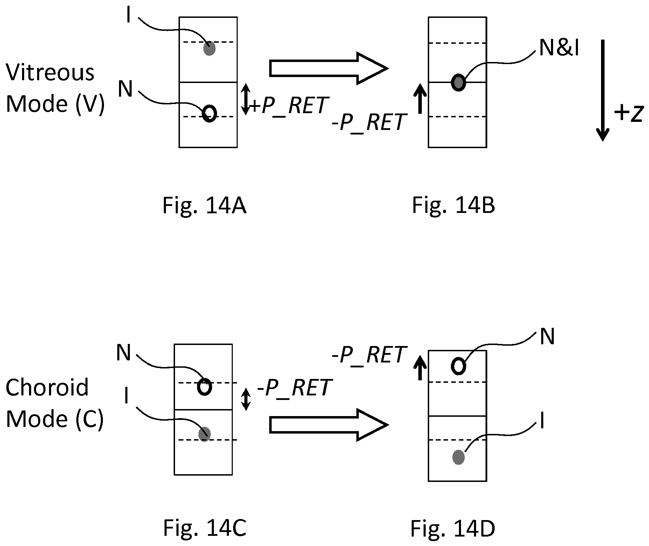

Option 2: Testing whether the predicted orientation is correct by moving the initial view of the retinal image by the amount $+/-P_{RET}$ (the distance of the visible image of the retina 127 from the ZDL) in a predetermined direction (+/- z). FIGs. 13 provide a schematic for a test move of $+P_{RET}$, whereas FIGs. 14 provide a schematic for test move of $-P_{RET}$.

Option 3: Testing whether the predicted orientation is correct by making a combination of moving by both $P_{RET}$ and $R_{DEST}$. Examples are shown in FIGs. 10C and 10D.

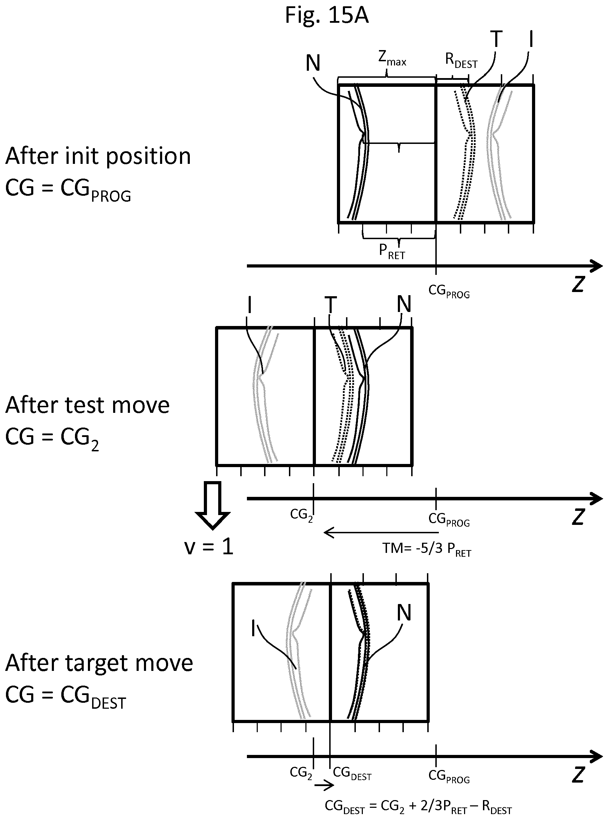

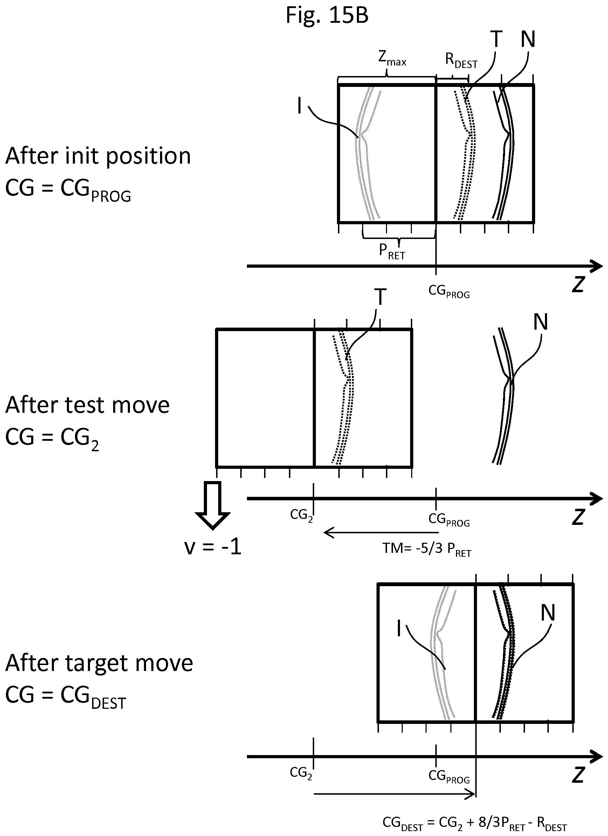

Option 4: Testing whether the predicted orientation is correct by moving the initial view of the retinal image by any other arbitrary distance in either predetermined direction. Examples are shown in Figs. 15A and 15B.

FIGs. 11-14 show a schematic depicting how the image of the retina 127 will move on the tomogram, the movement of the image of the retina 127 being dependent on movement of the reference mirror 132-4 in the reference arm R of the OCT apparatus 200. The drawings on the left (labelled A and C) show the predicted initial orientation S110. The drawings on the right (labelled B and D) show the arrangement after performing the test move S121.

The user is presented with a tomogram that corresponds to the lower half of the image (dashed boxes in FIGs. 4A and 4B, which corresponds to the lower halves of the tomograms shown in FIGs. 11-14). As shown in FIG. 4C, the ZDL is shown at the top of the visible tomogram and the position has a positive value (note that the z-axis is presented to be vertically downwards).

## Moving by $R_{DEST}$ (Option 1)

FIGs. 11A, 11B, 12A and 12B depict how the tomogram will change if the predicted orientation is the vitreous mode V (with the normal image N in the bottom, visible part of the tomogram and the inverted image I in the top, hidden part of the tomogram). FIGs. 11C, 11D, 12C and 12D depict how the tomogram will change if the predicted orientation is the choroid mode C (with the normal image in the top, hidden part of the tomogram).

FIGs. 11 show how the tomogram will change if the reference mirror 132-4 (and therefore the normal image of the retina 127) is moved by $-R_{DEST}$. If the orientation is in the vitreous mode V (FIGs. 11A and 11B), the position of the normal image N of the retina 127 changes from $+P_{RET}$ to $+P_{RET}-R_{DEST}$, causing the normal image N of the retina 127 to move towards the ZDL by $-R_{DEST}$. If the initial view is in the choroid mode C (FIG. 11C and 11D), then the position of the normal image N of the retina 127 changes from $-P_{RET}$ to $-P_{RET}-R_{DEST}$ causing the view of the inverted image I retina 127 also to move away from the ZDL by $+R_{DEST}$ (but in the visible part of the tomogram). As a consequence, by moving the normal image N by $-R_{DEST}$, the user can establish whether the initialisation view was in the choroid mode C or the vitreous mode V.

FIGs. 12 show how the tomogram will change if the reference mirror 132-4 (and therefore the normal image of the retina 127) is moved by $+R_{DEST}$. If the orientation is in the vitreous mode V (FIGs. 12A and 12B), the position of the normal image N of the retina 127 changes from $+P_{RET}$ to $+P_{RET}+R_{DEST}$, causing the view of the normal image N of the retina 127 to move away from the ZDL by $+R_{DEST}$. If the initial view is in the choroid mode C (FIGs. 12C and 12D), then the position of the image of the retina 127 changes from $-P_{RET}$ to $-P_{RET}+R_{DEST}$, causing the inverted image of the retina 127 to move towards the ZDL by $-R_{DEST}$. As a consequence, by moving the normal image N by $+R_{DEST}$, the user can establish whether the initialisation view was in the choroid mode C or the vitreous mode V.

After moving by $+/-R_{DEST}$ in step S121, the predicted orientation is obtained. However, the desired view is not obtained (NO in step S122). Depending upon whether the initial view was in the choroid mode C or the vitreous V, it may be possible to achieve the desired view by performing a target move of $+/-P_{RET}$ in step S123.

Options from 2 to 4 can be explained by two equations that define a test move that brings the zero delay line to position $CG_2$, and a target move that brings the zero delay line (or coherence gate as it is called in US 2013/0182218) to position $CG_{DEST}$. A mode (vitreous or choroid) is selected as being the desired viewing mode. As a result of the initialisation (and the zero delay line being at initial position $CG_{PROG}$), the image is at a position $P_{RET}$. The test move is intended to move the zero delay line in a specific direction by a specific distance. This specific distance is defined as a fraction of $P_{RET}$ by:

$$\mathrm{DIST} = w \star \alpha \star P_{RET},$$

which is a relative shift compared to the initial position $CG_{PROG}$ of the zero delay line.

Parameter w can be $+/-1$. By using this parameter, a choice is made as to whether the test move is to be positive or negative. If w is $+1$, the position of the zero delay line in the test move will be to increase it (toward the back of the retina 127). On the other hand, if w is $-1$, the position of the zero delay line in the test move will be decreased.

After the test move, zero delay line position will change to:

$$CG_2 = CG_{PROG} + w*\alpha*P_{RET}$$

Once the orientation has been determined having made the test move, the calculation of the target move typically involves undoing the test move (especially if the arbitrary test move sent the retinal image in the wrong direction) and doing a target move by $m*R_{DEST}$. Parameter m is related to the desired orientation of the image and is equal to -1 for vitreous orientation V and equal to +1 for choroid orientation C; m can be, for example, pre-selected by operator of the device, or it can be pre-determined by the system. Thus, zero delay line position will be set to destination location $CG_{DEST}$ by using formula:

$$CG_{DEST} = CG_2 - w*(\alpha-v)*P_{RET} + m*R_{DEST}$$

Depending on parameters w, $\alpha$ and v, we can obtain several options for the test moves. Table 1 summarizes parameter definitions for options 2 to 4.

**Table 1. Definitions of parameters w, $\alpha$ and v**

|  | w | $\alpha$ | v |
|---|---|---|---|
| Option 2 | w = +/-1 | 1 | +1: ZDL +/- $P_{RET}$ <br> -1: out of range |
| Option 3 | w = m | $1+R_{DEST}/P_{RET}$ | +1 : $R_{DEST}$ +/- $P_{RET}$ <br> -1: out of range |
| Option 4 | w = +/-1 | $\alpha_{min} < \alpha < \alpha_{max}$ | +1: ZDL +/- $Z_{MAX}$. <br> -1: out of range |

The parameter v is a result of observing the tomogram after the test move. If after the test move the image has moved out of range (e.g. FIG. 10D), then v takes the value of -1. If after the test move the image has moved to the position indicated in Table 1, then v takes the value of +1.

**Moving by $P_{RET}$ (Option 2)**

Table 1 shows that for option 2, parameter $\alpha$ = 1, for which the following equations are obtained to describe the test move and the target move:

$$CG_2 = CG_{PROG} + w*P_{RET}$$

$$CG_{DEST} = CG_2 - w*(1-v)*P_{RET} + m*R_{DEST}$$

The retinal image condition after the test move will be defined by parameter v which becomes +1 in case of retinal image is observed at range of ZDL to $P_{RET}$. If the retinal image is out of this range, parameter v becomes -1.
Description of the relative movement of the ZDL and the image can refer to either movement of the ZDL or movement of the image. If, looking at the figures, you imagine the ZDL to be fixed, this relative movement can be defined to take a positive value if the image moves in the positive direction of the z-axis (downwards in FIGs. 4 and 11-14; towards the right in FIGs. 10 and 15). Equivalently, considering the image to be fixed, this relative movement can be viewed as a positive value if the ZDL moves in the negative direction of the z-axis (upwards in FIGs. 4 and 9-12; towards the left in FIG. 14). It should be remembered that these two representations correspond to the same technical effect caused by movement of the reference mirror 132-4.
FIGs. 13A, 13B, 14A and 14B depict how the tomogram will change if the predicted orientation is the vitreous mode V (with the normal image N in the bottom, visible part of the tomogram and the inverted image I in the top, hidden part of the tomogram). FIGs. 13C, 13D, 14C and 14D depict how the tomogram will change if the predicted orientation is the choroid mode C (with the normal image N in the top, hidden part of the tomogram).
FIGs. 13 show how the tomogram will change if the zero delay line is moved in the -z direction by an amount of $-P_{RET}$, which corresponds to moving the normal image of the retina 127 in the +z direction by an amount of $+P_{RET}$.

This corresponds to a parameter value of w = -1. If the orientation is in the vitreous mode V (FIG. 13A and 13B), the position of the image of the retina 127 changes from $+P_{RET}$ to $+2P_{RET}$, which causes the normal image N of the retina 127 to move out of the range of ZDL +/- $P_{RET}$, thus v = -1, and the target move is defined as

$$CG_{DEST} = CG_2 + 2*P_{RET} + m*R_{DEST}$$

If the initial view is in the choroid mode C (FIGs. 13C and 13D), then the position of the normal image N of the retina 127 changes from $-P_{RET}$ to zero, causing an overlap N&I of the normal image N of the retina 127 in the vicinity of the ZDL. This condition would give v = 1 and the target move would be defined as

$$CG_{DEST} = CG_2 + m*R_{DEST}$$

As a consequence, by moving the retina 127 by $+P_{RET}$, the user can establish whether the initialisation view was in the choroid mode C or the vitreous mode V by looking at the resultant position of the image after the test move.
FIGs. 14 show how the tomogram will change if the reference mirror 132-4 increases its position (w = 1) and therefore the normal image of the retina 127 is moved by $-P_{RET}$. If the orientation is in the vitreous mode V (FIGs. 14A and 14B), the position of the normal image N of the retina 127 changes from $+P_{RET}$ to zero, causing an overlap N&I of the normal image of the retina 127 and the inverted image of the retina 127 in the vicinity of the ZDL thus v = 1 and target move will be given by formula $CG_{DEST} = CG_2 + m*R_{DEST}$.
If the initial view is in the choroid mode C (FIGs. 14C and 14D), then the position of the normal image N of the retina 127 changes from $-P_{RET}$ to $-2P_{RET}$, which could cause the inverted image I of the retina 127 to move out of the observed region so that the inverted image I can no longer be viewed within the range ZDL +/- $P_{RET}$, thus v becomes -1 and target move would be given by $CG_{DEST} = CG_2 - 2*P_{RET} + m*R_{DEST}$. As a consequence, by moving the retina 127 by $-P_{RET}$, the user can establish whether the initialisation view was in the choroid mode C or the vitreous mode V.
After moving by $+/-P_{RET}$ in step S121, the predicted orientation is obtained. However, the desired view is typically not obtained (NO in step S122). Depending upon whether the initial view was in the choroid mode C or the vitreous V, it may be possible to achieve the desired view by selecting parameter m as +/-1 respectively in step S123.

**Moving by both $P_{RET}$ and $R_{DEST}$ (Option 3)**

Table 1 shows that for option 3, parameter $\alpha = 1 + R_{DEST}/P_{RET}$ and w = m, for which the following equations are obtained to describe the test move and the target move:

$$CG_2 = CG_{PROG} + m*(P_{RET} + R_{DEST})$$

$$CG_{DEST} = CG_2 - m*(1-v)*P_{RET}$$

An advantage of this option is that there is a chance that after the test move, the arrangement is in the desired orientation and the desired position. Advantageously, if this happens (YES in step S122), there is no need to calculate or perform the target move S123. This reduces the burden on the OCT apparatus 200 because fewer moves will need to be performed.
Parameter v corresponds to presence of the retinal image on the tomogram within the area defined by range $R_{DEST}$ +/- $P_{RET}$. If the retinal image is present at tomogram within this range, then v = 1. If the retinal image is out of the range, then v = -1.
FIGs. 10C and 10D shows two examples of moving the initial view of the retina 127 by a test move that comprises a combination of both $P_{RET}$ and $R_{DEST}$. In both of the examples, FIGs. 10C and 10D show the normal image N of the retina 127, the inverted image I of the retina 127, and the desired orientation T.
In the example shown in FIG. 10C, the test move S121 results in the image of the retina 127 being in the desired position $R_{DEST}$. Accordingly, this brings an end to the procedure, and step S120 is completed.
In the example shown in FIG. 10D, the test move results in the image of the retina 127 not being in the desired position. Accordingly, in order to complete step S120, the target move S123 is calculated and performed.
In the examples shown in FIGs. 10C and 10D, it is assumed that the test move decreases the zero delay line position (w = -1) and the desired orientation and the desired position T is the same, corresponding to the vitreous mode V (m = -1) at a position of $+R_{DEST}$.

In example shown in FIG. 10C, the initial view has the normal image N of the retina 127 in the choroid orientation C at a position $-P_{RET}$. The inverted image I is in the choroid orientation C at a position $+P_{RET}$. This means that to obtain the desired view T in the vitreous mode V, the user performs a move of the normal image N from $-P_{RET}$ to $+R_{DEST}$. The test move S121 that is performed corresponds to $-P_{RET}-R_{DEST}$. As a consequence, the target move S123 is not calculated or performed.

In the example shown in FIG. 10D, the initial view has the normal image N of the retina 127 in the vitreous orientation V at a position $+P_{RET}$. The inverted image I is in the vitreous orientation V at a position $-P_{RET}$. This means that to obtain the desired view T in the vitreous mode V, the user needs to perform a move of the normal image N from $+P_{RET}$ to $+R_{DEST}$. Therefore, the resultant move is $+P_{RET}-R_{DEST}$. However, the user has performed the test move S121 of $-P_{RET}-R_{DEST}$. As a consequence, the normal image N of the retina 127 has moved from $+P_{RET}$ to a position of $+2P_{RET}+R_{DEST}$, causing the normal image N of the retina 127 to move out of the tomogram (thus v = -1). This information can be used to determine that the initial orientation was in the vitreous mode V, and so the required target move S123 to move the retinal image to the correct position (here in the vitreous mode $R_{DEST}$ from the ZDL) can be calculated and performed.

**Moving by an arbitrary distance (Option 4)**

The distance by which the retinal image is moved in the test move can be an arbitrary amount provided that the user can determine a) the direction that the image of the retina 127 has moved as a result and b) the orientation (vitreous or choroid) of the image. Accordingly, the specific distance should be sufficiently large for it to be possible to detect the distance moved by the image of the retina 127. Also, the arbitrary distance should be sufficiently small to ensure that the image of the retina 127 does not leave the view for at least one of the possible orientations.

Parameter $\alpha$ is limited by certain constraints:

$$\alpha_{min} \; = \; |1 \; - \; Z_{MAX}/(2*P_{RET})| \; + \; Z_{MAX}/(2*P_{RET})$$

in order to make sure that after the test move at step S121, the retinal image will be arranged in the opposite orientation from the initial position (hence the preference for the initialisation to place the zero delay line at the opposite orientation from the desired one); and

$$\alpha_{max} \; = \; Z_{MAX}/P_{RET} \; + \; 1,$$

to make sure that after step S121 the retinal image will be visible, and therefore will not be moved out of the imaging range $Z_{MAX}$. Brackets $|\,.\,|$ denotes a module operator.

Thus, $\alpha$ can be selected from range:

$$\alpha_{min} \; < \; \alpha \; < \; \alpha_{max}$$

In practice, it is advantageous to select $\alpha$ close to mid of the range, that is $\alpha_{opt} = (\alpha_{min} + \alpha_{max})/2$. Such $\alpha_{opt}$ will ensure that the test move is high enough to avoid uncertainties provided by involuntary eye movements.

An advantage of selecting the parameter $\alpha$ from range explained above is that it ensures the only two conditions can happen after the test move:

1) the retinal image is visible and it has opposite orientation comparing to the orientation before the test move, and
2) retina image is not visible thus it has the same orientation as orientation before the test move.

Thus, to determine parameter v, it is enough to determine presence or absence of the image within the entire tomogram rather than confirming the retinal image at certain range, as in option 2 or 3. This makes the process easier. However, disadvantage is that it would always require to perform the target move, because selecting $\alpha$ from defined range will not give retinal image position at $R_{DEST}$ anymore.

Defining the direction of movement of the zero delay line in the test move is explained below. For example, a negative w = -1 may be selected for the vitreous mode and a positive w = +1 may be selected for the choroid mode. This way, with the general movement of the zero delay line more likely to be towards the vitreum or choroidea respectively, giving the respective desired mode. Once the desired orientation is selected in the system, the algorithm used to control the movement of the reference mirror 132-4 (for example) will select a w = -1 which will mean that in the test move, the zero delay line will decrease its optical pathway, bringing it more likely into the vitreum. During the adjustment of the zero delay line, there are two possible conditions of the retinal image after the test move, as discussed above.

FIGs. 15A and 15B show examples of moving the retinal image by an arbitrary distance. Both of these drawings demonstrate how the position of the image on the tomogram is modified. The three images are consistent with the images shown in FIGs. 10. The top drawing shows how the tomogram will appear after the position of the object has been initialised by step S110. The middle drawing shows how the tomogram will appear after the test move of step S121. The bottom drawing shows how the tomogram will appear after the target move has been performed according to step S123. The positive z axis is show increasing towards the right of these drawings. Typically, the user is presented with the right hand side of the tomogram image.

FIG. 15A shows an initial choroid mode C, for which the inverted image I is displayed on the right-hand side of the tomogram. The desired image T is shown in the vitreous mode V. After the test move, the normal image N appears in the correct side of the tomogram, in the vitreous mode V, although the position is not correct. After the target move S123 has been performed, the desired image T is displayed.

FIG. 15B shows an initial vitreous mode V, for which the normal image N is displayed on the right-hand side of the tomogram. The desired image T is shown in the vitreous mode V. After the test move, the normal image N has moved off the tomogram, and so no image is displayed. After the target move S123 has been performed, the desired image T is displayed.

For the examples shown in FIGs. 15A and 15B, the ZDL is moved with respect to the z-axis, with the normal image N maintaining a constant position value on the z-axis.

**Position Correction**

In practical realization of the process, the involuntary eye movements should be taken into consideration. For this reason, the last process S130 of retinal image position correction might be required, although it is not mandatory.

FIG. 8 outlines a method for correcting the position of the retina 127 on the tomogram according to process S130.

In step S131, the position of the retina 127 relative to the optical pathway in the sample arm S in the OCT apparatus 200 is once again measured In step S132, the current position of the image of the retina ($P_{RET}$) is compared with the desired position of the image of the retina ($R_{DEST}$) . The position error ($P_{ERR}$) is defined as the difference between the measured position and the desired position ($P_{ERR} = P_{RET} - R_{DEST}$) .

In step S133, it is determined whether the error of the position is within a required precision ($\varepsilon$), by assessing whether $P_{ERR} < \varepsilon$. If $P_{ERR} < \varepsilon$ (YES in step S133), then the precision is sufficient, and so the process ends. However, if $P_{ERR} >= \varepsilon$ (NO in step S133) then the process continues to step S134. It is not necessary to perform the test in step S133 if the correction is performed regardless of the error value.

In step S134, the reference mirror 132-4 is moved so that the image of the retina 127 is corrected to have the desired position on the tomogram. This involves moving the retina 127 by an amount $P_{ERR}$. The process ends once the reference mirror 132-4 has moved the tomogram to the correct position relative to the retinal image.

There are a several ways in which the current position $P_{RET}$ of the retinal image on the tomogram can be determined.

At first, the set of A-scans is selected. In particular, all A-scans can be taken into account. Next, noise on selected A-scans is removed by using a binary threshold. Next, a position of the retina 127 on each A-scan is calculated as the geometric center of pixels with value larger than 0 making up the A-scan. The position of the retina 127 on the tomogram is the averaged value of the geometric center calculated for the A-scans. The position is expressed in units of pixels. In order to convert it to micrometers, the position in pixels is multiplied by a certain scaling factor. The scaling factor corresponds to the size of a single pixel of a tomogram in the A-scan direction and it mainly depends on construction of a spectrometer used in the SD-OCT device. The scaling factor can be also determined experimentally by using a mirror as an object and creating two tomogram images corresponding to two different positions of the mirror. Knowing the two positions of the mirror and finding the location of the surface image on the tomogram, one can find the scaling factor.

**Alternative implementations**

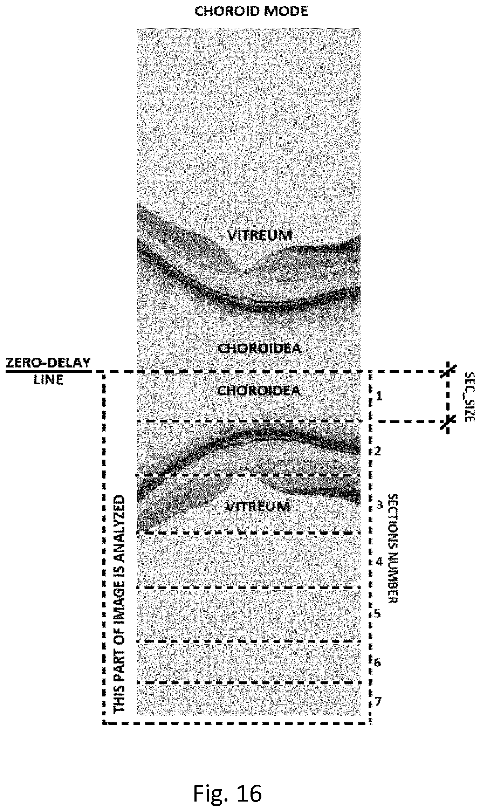

A technique for determining the position is to measure the distance between the ZDL and the part of the retinal image that is to be viewed on the tomogram. FIG. 16 shows an alternative technique for moving the zero delay line relative to the retinal image, which is to divide the tomogram into segments and to determine which segment contains the part of the retinal image that is to be viewed on the tomogram.

There are several ways in which the position of the retinal image can be moved on the tomogram.

A first technique is to move the reference mirror 132-4 by a specific amount over a continuous range.

A second technique is to move the reference mirror 132-4 by a specific number of discrete steps, wherein the tomogram is divided into sections (sections numbered 1 to 7) corresponding to the discrete steps that may be equal in size (FIG. 16). For example, the sections could correspond to a distance of the object equal to 1 micrometre.

A third technique would be to move the object itself with respect to the OCT apparatus 200 (or to move to the whole OCT apparatus), although this is likely to be unreliable.

Other ways of adjusting the sample and reference optical paths are available, for example, as provided in PL 218325 B1.

If a high degree of accuracy is not required for the position of the image on the tomogram, the target position could be determined roughly in step S123. Also, the initial orientation of the retina 127 could be determined in step 100 by searching through the whole possible range of optical path lengths and analysing the OCT images while the optical path length changes. The optical path length starts from one of the possible limit positions (see FIG. 6B), optionally starting from the opposite limit position relative to the ZDL than the position of the desired orientation. The OCT image analysis can be done, for example, by using an algorithm that implements a signal to noise ratio (SNR). Thus, the calculation of possible positions of the image of the retina 127 on the tomogram can be avoided. Instead, the tomogram can be divided into N sections (as shown in FIG. 16) of size SEC_SIZE and the SNR can be calculated for each section. If the SNR for a particular section is greater than a predetermined value, then this indicates that the image of the retina 127 is present in this section of the tomogram.

The accuracy of setting the image on a tomogram would be defined by SEC_SIZE (section size) which is given by equation:

$$\text{SEC\_SIZE} = Z_{MAX} \: / \: N$$

For example, if $Z_{MAX}$ = 2.1 mm and N=7, SEC_SIZE (and accuracy) would be 2.1mm/7 = 0.3mm.

When using sections, a means for changing the optical path length (e.g. reference mirror 132-4), could be driven in step-manner instead of a continuous move, thus improving convergence time. This is especially advantageous in the case of using other optical delay line systems instead of a moving mirror, e.g. grating-based phase control optical delay line (see US6111645A).

The above examples can also be realised by a computer of a system or apparatus (or devices such as a CPU or MPU) that reads out and executes a program recorded on a memory device to perform the functions of the above-described examples, and by a method, the steps of which are performed by a computer of a system or apparatus by, for example, reading out and executing a program recorded on a memory device to perform the functions of the above-described examples. For this purpose, the program is provided to the computer for example via a network or from a recording medium of various types serving as the memory device (e.g., computer-readable medium).

**Claims**

1. A method of an optical coherence tomography apparatus (200) for positioning on a tomogram an image of a retina (127) of an eye (107), the method comprising:

   initialising a reference optical path length of the optical coherence tomography apparatus (200) so that an image of the retina (127) can be viewed on the tomogram at an initial position (S110), by setting the optical coherence tomography apparatus (200) into one of a vitreous mode of operation and a choroid mode of operation;
   performing a test move (S121) by changing the reference optical path length in a first direction by a first distance;
   observing a change in the tomogram of the retina (127) as a result of the test move;
   determining, from the observation, whether an initial orientation of the image of the retina (127) was a normal orientation (N) or an inverted orientation (I); and
   performing a target move (S123) by changing the reference optical path length of the optical coherence tomography apparatus (200) so that the image of the retina (127) can be placed at a desired position within the tomogram based on the initial orientation of the image of the retina (127);
   wherein in the vitreous mode (V) of operation the optical coherence tomography apparatus (200) is configured to position an image of the eye in the normal orientation (N) with an image of the vitreous of the eye located closer to a zero delay line (ZDL) than an image of the choroidea of the eye, and
   wherein in the choroid mode (C) of operation the optical coherence tomography apparatus (200) is configured to position an image of the eye in the inverted orientation (I) with an image of the choroid of the eye located closer to a zero delay line (ZDL) than an image of the vitreous of the eye.

2. A method of an optical coherence tomography apparatus (200) according to claim 1, wherein the observing step comprises observing a direction in which the retina (127) has moved in the tomogram or observing whether or not the test move leaves the image of the retina (127) within the tomogram.

3. A method of an optical coherence tomography apparatus (200) according to claim 1 or claim 2, wherein:
   the position of the image of the retina (127) in the tomogram is determined by measuring the distance of the image of the retina (127) from a zero delay line (ZDL) of the tomogram.

4. A method of an optical coherence tomography apparatus (200) according to claim 3, wherein the test move (S121) comprises:
   selecting the first direction and the first distance according to:

$$CG_2 = CG_{PROG} + \alpha * R_{DEST}$$

wherein $CG_2$ is the resulting position of the zero delay line (ZDL) after the test move (S121); $CG_{PROG}$ is the initial position of the zero delay line (ZDL); $R_{DEST}$ is the desired position of the retina (127) within the tomogram relative to the zero delay line (ZDL); and $\alpha$ is a parameter that may be positive to increase the reference optical path length or negative to reduce the reference optical path length.

5. A method of an optical coherence tomography apparatus (200) according to claim 3, wherein the test move (S121) comprises:
   selecting the first direction and the first distance according to:

$$CG_2 = CG_{PROG} + w * P_{RET}$$

wherein $CG_2$ is the resulting position of the zero delay line (ZDL) after the test move (S121); $CG_{PROG}$ is the initial position of the zero delay line (ZDL); $P_{RET}$ is the initial position of the retina (127) within the tomogram relative to the zero delay line (ZDL); and w is a parameter that may be positive to increase the reference optical path length or negative to reduce the reference optical path length.

6. A method of an optical coherence tomography apparatus (200) according to claim 3, wherein the test move (S121) comprises:
   selecting the first direction and the first distance according to:

$$CG_2 = CG_{PROG} + m * (P_{RET} + R_{DEST})$$

wherein $CG_2$ is the resulting position of the zero delay line (ZDL) after the test move (S121); $CG_{PROG}$ is the initial position of the zero delay line (ZDL); $P_{RET}$ is the initial position of the retina (127) within the tomogram relative to the zero delay line (ZDL); $R_{DEST}$ is the desired position of the retina (127) within the tomogram relative to the zero delay line (ZDL); and m is a parameter that may be positive to increase the reference optical path length or negative to reduce the reference optical path length.

7. A method of an optical coherence tomography apparatus (200) according to claim 3, wherein the test move (S121) comprises:
   selecting the first direction and the first distance according to:

$$CG_2 = CG_{PROG} + w * \alpha * P_{RET}$$

wherein $CG_2$ is the resulting position of the zero delay line (ZDL) after the test move (S121); $CG_{PROG}$ is the initial position of the zero delay line (ZDL); $P_{RET}$ is the initial position of the retina (127) within the tomogram relative to the zero delay line (ZDL); $\alpha$ is a parameter that takes a value between $\alpha_{min} = |1 - Z_{MAX/} (2 * P_{RET})| + Z_{MAX/} (2 * P_{RET})$ and $\alpha_{max} = Z_{MAX}/P_{RET} + 1$, where $Z_{MAX}$ is an imaging range of the optical coherence tomography apparatus; and w is a parameter that may be positive to increase the reference optical path length or negative to reduce the reference optical path length.

8. A method of an optical coherence tomography apparatus (200) according to any one of claims 1 to 7, wherein if the position of the image of the retina (127) within the tomogram after the test move corresponds to a desired position, then it is determined that it is not necessary to perform the target move.

9. A method of an optical coherence tomography apparatus (200) according to any preceding claim, wherein initialising (S110) the reference optical path length comprises:
calculating an initial reference optical path length that is adapted to give rise to an initial orientation of the image of the retina (127) at the initial position, wherein the initial orientation is different from the orientation (T) corresponding to the set mode of operation.

10. A method of an optical coherence tomography apparatus (200) according to any preceding claim, wherein initialising (S110) the reference optical path length comprises the steps of:

setting the reference optical path length in the optical coherence tomography apparatus (200) at a limit position of the range of movement of a reference mirror(S113B); and
changing the reference optical path length (S114B) until the image of the retina (127) is visible on the tomogram (S115B).

11. A method of an optical coherence tomography apparatus (200) according to any of claims 1 to 10, wherein:

the object is a retina (127) of an eye (107); and
initialising the reference optical path length comprises estimating a position of the retina by determining an axial length (D) of the eye (107) based upon a refraction error (F) of the eye (107).

12. A method of an optical coherence tomography apparatus (200) according to any preceding claim,
wherein estimating the position of the retina comprises measuring a position (w) of an anterior segment of the eye using light reflected from the anterior segment of the eye and adding the determined axial length (D) of the eye measured using the refraction error (F) of the eye which is measured using a scanning laser ophthalmoscope.

13. A method of an optical coherence tomography apparatus (200) according to any preceding claim, further comprising:

a subsequent step of measuring the position of the image of the retina (127) on the tomogram (S131);
comparing the desired position ($R_{DEST}$) of the image of the retina (127) with a measured position of the image of the retina (127) (S132); and
correcting the position of the retina (127) by changing the reference optical path length of the optical coherence tomography apparatus (200) so that an image of the retina (127) is moved from the measured position to the desired position (S134).

14. A method of an optical coherence tomography apparatus (200) according to any preceding claim, further comprising:

dividing the tomogram into a plurality of sections (1-7); and
determining the position of the image of the retina (127) by identifying a section of the tomogram in which the image of the retina (127) is positioned.

15. A method of an optical coherence tomography apparatus (200) according to claim 14, wherein dividing the tomogram into sections comprises:

dividing the tomogram into sections (1-7) that have equal length,
wherein the method further comprises:
modifying the optical path length in units related to the length of the sections.

16. A method of an optical coherence tomography apparatus (200) according to any one of claims 1 to 15, wherein the target move (S123) is performed based on the change in the tomogram of the retina (127) as a result of the test move, the initial position and the desired position of the image of the retina (127) within the tomogram, and a mode of operation,
wherein the mode of operation is one of a vitreous mode of operation (V) and a choroid mode of operation (C).

17. An optical coherence tomography apparatus (200) for positioning on a tomogram an image of a retina (127) of an eye (107), the optical coherence tomography apparatus (200) comprising:

initialising means (131-3) adapted to initialise a reference optical path length of the optical coherence tomography apparatus (200) so that an image of the retina (127) can be viewed on the tomogram at an initial position ($P_{RET}$), by setting the optical coherence tomography apparatus (200) into one of a vitreous mode of operation and a choroid mode of operation;

changing means (132-4) configured to perform a test move (S121) by changing the reference optical path length in a first direction by a first distance;

observing means (928) configured to observe a change in the tomogram of the retina (127) as a result of the test move; and

orientation determining means (925) configured to determine, from the observation, whether an initial orientation of the image of the retina (127) was a normal orientation (N) or an inverted orientation (I) ;

wherein the changing means (132-4) is configured to perform a target move (S123) by changing the reference optical path length of the optical coherence tomography apparatus (200) so that the image of the retina (127) can be placed at a desired position within the tomogram based on the initial orientation of the image of the retina (127);

wherein in the vitreous mode (V) of operation the optical coherence tomography apparatus (200) is configured to position an image of the eye in the normal orientation (N) with an image of the vitreous of the eye located closer to a zero delay line (ZDL) than an image of the choroidea of the eye, and

wherein in the choroid mode (C) of operation the optical coherence tomography apparatus (200) is configured to position an image of the eye in the inverted orientation (I) with an image of the choroid of the eye located closer to a zero delay line (ZDL) than an image of the vitreous of the eye.

18. An optical coherence tomography apparatus (200) according to claim 17, further comprising:

an object-side optical path (S) between the retina (127) and an interferometer; and
a reference-side optical path (R) between a reference mirror (132-4) and the interferometer;
wherein the reference optical path length that is adjusted is an optical path length of the reference-side optical path (R).

19. An optical coherence tomography apparatus (200) according to claim 17, further comprising:

an object-side optical path (S) between the retina (127) and an interferometer; and
a reference-side optical path (R) between a reference mirror (132-4) and the interferometer;
wherein the reference optical path length that is adjusted is an optical path length of the object-side optical path (S).

20. An optical coherence tomography apparatus (200) according to any one of claims 17 to 19, further comprising:

a light source (101) for emitting a low-coherence light;
an interferometer for generating an interference light by splitting the emitted low-coherence light into a sample light to be transmitted in a sample pathway (S) to a retina (127) to be measured and into a reference light to be transmitted in a reference pathway (R) to a reference object, and superposing the sample light having passed through the retina (127) to be measured and the reference light having passed through the reference object,
a detector for detecting the generated interference light; and
an image-forming means for forming an image of the retina (127) to be measured on a tomogram based on the result of detection by the detector,
wherein the changing means (132-4) is configured to change a difference in optical path length between the sample light and the reference light based on the determination result of the orientation determining means (925).

21. An optical coherence tomography apparatus (200) according to any one of claims 17 to 20, wherein the target move (S123) is performed based on the change in the tomogram of the retina (127) as a result of the test move, the initial position and the desired position of the image of the retina (127) within the tomogram, and a mode of operation, wherein the mode of operation is one of a vitreous mode of operation (V) and a choroid mode of operation (C) .

22. A program that, when implemented by an optical coherence tomography apparatus (200), causes the optical coherence tomography apparatus (200) to perform a method according to any one of claims 1 to 16.

23. A storage medium storing a program according to claim 22.

**Patentansprüche**

1. Verfahren einer Vorrichtung (200) für optische Kohärenztomographie zum Positionieren eines Bildes einer Netzhaut (127) eines Auges (107) auf einem Tomogramm, wobei das Verfahren umfasst:

   Initialisieren einer Referenzstrahlengangänge der Vorrichtung (200) für optische Kohärenztomographie, sodass ein Bild der Netzhaut (127) auf dem Tomogramm an einer Initialposition betrachtet werden kann (S110), durch Einstellen der Vorrichtung für optische Kohärenztomographie in einen Glaskörper-Betriebsmodus oder einen Aderhaut-Betriebsmodus;
   Durchführen einer Testbewegung (S121) durch Ändern der Referenzstrahlengangänge in einer ersten Richtung um einen ersten Abstand;
   Beobachten einer Änderung der Netzhaut (127) im Tomogramm als ein Ergebnis der Testbewegung;
   Bestimmen, aufgrund der Beobachtung, ob es sich bei einer Initialorientierung des Bildes der Netzhaut (127) um eine normale Orientierung (N) oder eine invertierte Orientierung (I) handelte; und
   Durchführen einer Zielbewegung (S123) durch Ändern der Referenzstrahlengangänge der Vorrichtung (200) für optische Kohärenztomographie, sodass das Bild der Netzhaut (127) an einer gewünschten Position innerhalb des Tomogramms platziert werden kann, basierend auf der Initialorientierung des Bildes der Netzhaut (127); wobei im Glaskörper-Betriebsmodus (V) die Vorrichtung (200) für optische Kohärenztomographie konfiguriert ist, ein Bild des Auges in der normalen Orientierung (N) mit einem Bild des Glaskörpers des Auges näher an einer Nullverzögerungslinie (ZDL) als ein Bild der Aderhaut des Auges zu positionieren, und wobei im Aderhaut-Betriebsmodus (C) die Vorrichtung (200) für optische Kohärenztomographie konfiguriert ist, ein Bild des Auges in der invertierten Orientierung (I) mit einem Bild der Aderhaut des Auges näher an einer Nullverzögerungslinie (ZDL) als ein Bild des Glaskörpers des Auges zu positionieren.

2. Verfahren einer Vorrichtung (200) für optische Kohärenztomographie nach Anspruch 1, wobei der Beobachtungsschritt das Beobachten einer Richtung umfasst, in welche sich die Netzhaut (127) im Tomogramm bewegt hat, oder das Beobachten umfasst, ob die Testbewegung das Bild der Netzhaut (127) innerhalb des Tomogramms belässt oder nicht.

3. Verfahren einer Vorrichtung (200) für optische Kohärenztomographie nach Anspruch 1 oder Anspruch 2, wobei:
   die Position des Bildes der Netzhaut (127) im Tomogramm durch Messen des Abstands des Bildes der Netzhaut (127) von einer Nullverzögerungslinie (ZDL) des Tomogramms bestimmt wird.

4. Verfahren einer Vorrichtung (200) für optische Kohärenztomographie nach Anspruch 3, wobei die Testbewegung (S121) umfasst:
   Auswählen der ersten Richtung und des ersten Abstands gemäß:

$$CG_2 \ = \ CG_{PROG} \ + \ \alpha * R_{DEST}$$

   wobei $CG_2$ die resultierende Position der Nullverzögerungslinie (ZDL) nach der Testbewegung ist (S121); $CG_{PROG}$ die Initialposition der Nullverzögerungslinie (ZDL) ist; $R_{DEST}$ die gewünschte Position der Netzhaut (127) innerhalb des Tomogramms relativ zur Nullverzögerungslinie (ZDL) ist; und $\alpha$ ein Parameter ist, der positiv sein kann, um die Referenzstrahlengangänge zu vergrößern, oder negativ sein kann, um die Referenzstrahlengangänge zu verkleinern.

5. Verfahren einer Vorrichtung (200) für optische Kohärenztomographie nach Anspruch 3, wobei die Testbewegung (S121) umfasst:
   Auswählen der ersten Richtung und des ersten Abstands gemäß:

$$CG_2 \ = \ CG_{PROG} \ + \ w * P_{RET}$$

   wobei $CG_2$ die resultierende Position der Nullverzögerungslinie (ZDL) nach der Testbewegung ist (S121); $CG_{PROG}$ die Initialposition der Nullverzögerungslinie (ZDL) ist; $P_{RET}$ die Initialposition der Netzhaut (127) innerhalb des Tomogramms relativ zur Nullverzögerungslinie (ZDL) ist; und w ein Parameter ist, der positiv sein kann, um die Referenzstrahlengangänge zu vergrößern, oder negativ sein kann, um die Referenzstrahlengangänge zu verkleinern.

6. Verfahren einer Vorrichtung (200) für optische Kohärenztomographie nach Anspruch 3, wobei die Testbewegung (S121) umfasst:
Auswählen der ersten Richtung und des ersten Abstands gemäß:

$$CG_2 = CG_{PROG} + m*(P_{RET} + R_{DEST})$$

wobei $CG_2$ die resultierende Position der Nullverzögerungslinie (ZDL) nach der Testbewegung ist (S121); $CG_{PROG}$ die Initialposition der Nullverzögerungslinie (ZDL) ist; $P_{RET}$ die Initialposition der Netzhaut (127) innerhalb des Tomogramms relativ zur Nullverzögerungslinie (ZDL) ist; **$R_{DEST}$** die gewünschte Position der Netzhaut (127) innerhalb des Tomogramms relativ zur Nullverzögerungslinie (ZDL) ist; und m ein Parameter ist, der positiv sein kann, um die Referenzstrahlenganglänge zu vergrößern, oder negativ sein kann, um die Referenzstrahlengangänge zu verkleinern.

7. Verfahren einer Vorrichtung (200) für optische Kohärenztomographie nach Anspruch 3, wobei die Testbewegung (S121) umfasst:
Auswählen der ersten Richtung und des ersten Abstands gemäß:

$$CG_2 = CG_{PROG} + w*\alpha*P_{RET}$$

wobei $CG_2$ die resultierende Position der Nullverzögerungslinie (ZDL) nach der Testbewegung ist (S121); $CG_{PROG}$ die Initialposition der Nullverzögerungslinie (ZDL) ist; $P_{RET}$ die Initialposition der Netzhaut (127) innerhalb des Tomogramms relativ zur Nullverzögerungslinie (ZDL) ist; $\alpha$ ein Parameter ist, der einen Wert zwischen $\alpha_{min} = |\,1 - Z_{MAX}/(2*P_{RET})\,| + Z_{MAX}/(2*P_{RET})$ und $\alpha_{max} = Z_{MAX}/P_{RET} + 1$ annimmt, wobei $Z_{MAX}$ ein Abbildungsbereich der Vorrichtung für optische Kohärenztomographie ist; und w ein Parameter ist, der positiv sein kann, um die Referenzstrahlengangänge zu vergrößern, oder negativ sein kann, um die Referenzstrahlengangänge zu verkleinern.

8. Verfahren einer Vorrichtung (200) für optische Kohärenztomographie nach einem der Ansprüche 1 bis 7, wobei, falls die Position des Bildes der Netzhaut (127) innerhalb des Tomogramms nach der Testbewegung einer gewünschten Position entspricht, bestimmt wird, dass die Zielbewegung nicht durchgeführt werden muss.

9. Verfahren einer Vorrichtung (200) für optische Kohärenztomographie nach einem der vorhergehenden Ansprüche, wobei das Initialisieren (S110) der Referenzstrahlengangänge umfasst:
Berechnen einer Initialreferenzstrahlengangänge, die angepasst wird, um eine Initialorientierung des Bildes der Netzhaut (127) an der Initialposition auszulösen, wobei die Initialorientierung verschieden von der dem eingestellten Betriebsmodus entsprechenden Orientierung (T) ist.

10. Verfahren einer Vorrichtung (200) für optische Kohärenztomographie nach einem der vorhergehenden Ansprüche, wobei ein Initialisieren (S110) der Referenzstrahlengangänge folgende Schritte umfasst:

Einstellen der Referenzstrahlengangänge in der Vorrichtung (200) für optische Kohärenztomographie an einer Grenzposition des Bewegungsbereichs eines Referenzspiegels (S113B); und
Ändern der Referenzstrahlengangänge (S114B), bis das Bild der Netzhaut (127) auf dem Tomogramm (S115B) sichtbar ist.

11. Verfahren einer Vorrichtung (200) für optische Kohärenztomographie nach einem der Ansprüche 1 bis 10, wobei:

das Objekt eine Netzhaut (127) eines Auges (107) ist; und
Initialisieren der Referenzstrahlengangänge das Schätzen einer Position der Netzhaut durch Bestimmen einer Axiallänge (D) des Auges (107) umfasst, basierend auf einem Brechungsfehler (F) des Auges (107).

12. Verfahren einer Vorrichtung (200) für optische Kohärenztomographie nach einem der vorhergehenden Ansprüche, wobei das Schätzen der Position der Netzhaut das Messen einer Position (w) eines vorderen Segments des Auges unter Verwendung von vom vorderen Segment des Auges reflektierten Lichts umfasst sowie Hinzufügen der bestimmten Axiallänge (D) des Auges, gemessen unter Verwendung des unter Verwendung eines Scanning-Laser-Ophthalmoskops gemessenen Brechungsfehlers (F) des Auges.

13. Verfahren einer Vorrichtung (200) für optische Kohärenztomographie nach einem der vorhergehenden Ansprüche, ferner umfassend:

einen nachfolgenden Schritt zum Messen der Position des Bildes der Netzhaut (127) auf dem Tomogramm (S131);
Vergleichen der gewünschten Position ($R_{DEST}$) des Bildes der Netzhaut (127) mit einer gemessenen Position des Bildes der Netzhaut (127) (S132); und
Korrigieren der Position der Netzhaut (127) durch Ändern der Referenzstrahlengganglänge der Vorrichtung (200) für optische Kohärenztomographie, so dass ein Bild der Netzhaut (127) von der gemessenen Position zur gewünschten Position bewegt wird (S134).

14. Verfahren einer Vorrichtung (200) für optische Kohärenztomographie nach einem der vorhergehenden Ansprüche, ferner umfassend:

Unterteilen des Tomogramms in mehrere Abschnitte (1-7); und
Bestimmen der Position des Bildes der Netzhaut (127) durch Identifizieren eines Abschnitts des Tomogramms, in dem das Bild der Netzhaut (127) positioniert ist.

15. Verfahren einer Vorrichtung (200) für optische Kohärenztomographie nach Anspruch 14, wobei das Unterteilen des Tomogramms in Abschnitte umfasst:

Unterteilen des Tomogramms in Abschnitte (1-7), die gleiche Länge aufweisen,
wobei das Verfahren ferner umfasst:
Modifizieren der Strahlengganglänge in Einheiten bezüglich der Länge der Abschnitte.

16. Verfahren einer Vorrichtung (200) für optische Kohärenztomographie nach einem der Ansprüche 1 bis 15, wobei die Zielbewegung (S123) basierend auf der Änderung der Netzhaut (127) im Tomogramm als ein Ergebnis der Testbewegung, der Initialposition und der gewünschten Position des Bildes der Netzhaut (127) innerhalb des Tomogramms, und einem Betriebsmodus durchgeführt wird,
wobei der Betriebsmodus ein Glaskörper-Betriebsmodus (V) oder ein Aderhaut-Betriebsmodus (C) ist.

17. Vorrichtung (200) für optische Kohärenztomographie zum Positionieren eines Bildes einer Netzhaut (127) eines Auges (107) auf einem Tomogramm, wobei die Vorrichtung (200) für optische Kohärenztomographie umfasst:

eine Initialisierungseinrichtung (131-3), die ausgebildet ist zum Initialisieren einer Referenzstrahlengganglänge der Vorrichtung (200) für optische Kohärenztomographie, sodass ein Bild der Netzhaut (127) auf dem Tomogramm an einer Initialposition (PRET) betrachtet werden kann, durch Einstellen der Vorrichtung (200) für optische Kohärenztomographie in einen Glaskörper-Betriebsmodus oder einen Aderhaut-Betriebsmodus;
eine Änderungseinrichtung (132-4), die konfiguriert ist zum Durchführen einer Testbewegung (S121) durch Ändern der Referenzstrahlengganglänge in einer ersten Richtung um einen ersten Abstand;
eine Beobachtungseinrichtung (928), die konfiguriert ist zum Beobachten einer Änderung der Netzhaut (127) im Tomogramm als ein Ergebnis der Testbewegung; und
eine Orientierungsbestimmungseinrichtung (925), die konfiguriert ist zum Bestimmen, aufgrund der Beobachtung, ob es sich bei einer Initialorientierung des Bildes der Netzhaut (127) um eine normale Orientierung (N) oder eine invertierte Orientierung (I) handelte; und
wobei die Änderungseinrichtung (132-4) konfiguriert ist zum Durchführen einer Zielbewegung (S123) durch Ändern der Referenzstrahlengganglänge der Vorrichtung (200) für optische Kohärenztomographie, sodass das Bild der Netzhaut (127) an einer gewünschten Position innerhalb des Tomogramms platziert werden kann, basierend auf der Initialorientierung des Bildes der Netzhaut (127);
wobei im Glaskörper-Betriebsmodus (V) die Vorrichtung (200) für optische Kohärenztomographie konfiguriert ist, ein Bild des Auges in der normalen Orientierung (N) mit einem Bild des Glaskörpers des Auges näher an einer Nullverzögerungslinie (ZDL) als ein Bild der Aderhaut des Auges zu positionieren, und
wobei im Aderhaut-Betriebsmodus (C) die Vorrichtung (200) für optische Kohärenztomographie konfiguriert ist, ein Bild des Auges in der invertierten Orientierung (I) mit einem Bild der Aderhaut des Auges näher an einer Nullverzögerungslinie (ZDL) als ein Bild des Glaskörpers des Auges zu positionieren.

18. Vorrichtung (200) für optische Kohärenztomographie nach Anspruch 17, ferner umfassend:

einen objektseitigen Strahlengang (S) zwischen der Netzhaut (127) und einem Interferometer; und
einen referenzseitigen Strahlengang (R) zwischen einem Referenzspiegel (132-4) und dem Interferometer;
wobei die Referenzstrahlenganglänge, die angepasst wird, eine Strahlenganglänge des referenzseitigen Strahlengangs (R) ist.

19. Vorrichtung (200) für optische Kohärenztomographie nach Anspruch 17, ferner umfassend:

einen objektseitigen Strahlengang (S) zwischen der Netzhaut (127) und einem Interferometer; und
einen referenzseitigen Strahlengang (R) zwischen einem Referenzspiegel (132-4) und dem Interferometer;
wobei die Referenzstrahlenganglänge, die angepasst wird, eine Strahlenganglänge des objektseitigen Strahlengangs (S) ist.

20. Vorrichtung (200) für optische Kohärenztomographie nach einem der Ansprüche 17 bis 19, ferner umfassend:

eine Lichtquelle (101) zum Emittieren von Licht mit niedriger Kohärenz;
ein Interferometer zum Erzeugen eines Interferenzlichts durch Aufteilen des emittierten Lichts mit niedriger Kohärenz in ein Probelicht, das in einem Probegang (S) zu einer zu messenden Netzhaut (127) übertragen werden soll und in ein Referenzlicht, das in einem Referenzgang (R) zu einem Referenzobjekt übertragen werden soll, und Überlagern des Probelichts, das die zu messende Netzhaut (127) passiert, und des Referenzlichts, das das Referenzobjekt passiert,
einen Detektor zum Detektieren des erzeugten Interferenzlichts; und
eine Bilderzeugungseinrichtung zum Erzeugen eines Bildes der zu messenden Netzhaut (127) auf einem Tomogramm, basierend auf dem Detektionsergebnis des Detektors,
wobei die Änderungseinrichtung (132-4) konfiguriert ist zum Ändern einer Differenz der Strahlenganglänge zwischen dem Probelicht und dem Referenzlicht basierend auf dem Bestimmungsergebnis der Orientierungsbestimmungseinrichtung (925).

21. Vorrichtung (200) für optische Kohärenztomographie nach einem der Ansprüche 17 bis 20, wobei die Zielbewegung (S123) basierend auf der Änderung der Netzhaut (127) im Tomogramm als ein Ergebnis der Testbewegung, der Initialposition und der gewünschten Position des Bildes der Netzhaut (127) innerhalb des Tomogramms, und einem Betriebsmodus durchgeführt wird,
wobei der Betriebsmodus ein Glaskörper-Betriebsmodus (V) oder ein Aderhaut-Betriebsmodus (C) ist.

22. Programm, das bei Implementierung durch eine Vorrichtung (200) für optische Kohärenztomographie diese dazu veranlasst, ein Verfahren nach einem der Ansprüche 1 bis 16 durchzuführen.

23. Speichermedium, das ein Programm nach Anspruch 22 speichert.

**Revendications**

1. Procédé destiné à un appareil de tomographie par cohérence optique (200) pour positionner, sur un tomogramme, une image d'une rétine (127) d'un oeil (107), le procédé comprenant les étapes consistant à :

initialiser une longueur de trajet optique de référence de l'appareil de tomographie par cohérence optique (200) de façon à pouvoir visualiser une image de la rétine (127) sur le tomogramme à une position initiale (S110), par une mise de l'appareil de tomographie par cohérence optique (200) dans l'un d'un mode de fonctionnement corps vitré et d'un mode de fonctionnement choroïde ;
exécuter un déplacement test (S121) par une modification, d'une première distance, de la longueur de trajet optique de référence dans une première direction ;
observer un changement dans le tomogramme de la rétine (127) en tant que résultat du déplacement test ;
déterminer, à partir de l'observation, si une orientation initiale de l'image de la rétine (127) est une orientation normale (N) ou une orientation inversée (I) ; et
exécuter un déplacement cible (S123) par une modification de la longueur de trajet optique de référence de l'appareil de tomographie par cohérence optique (200) de façon à pouvoir placer l'image de la rétine (127) à une position souhaitée à l'intérieur du tomogramme sur la base de l'orientation initiale de l'image de la rétine (127) ;
dans lequel, dans le mode de fonctionnement corps vitré (V), l'appareil de tomographie par cohérence optique (200) est configuré pour positionner une image de l'oeil dans l'orientation normale (N), une image du corps vitré de l'oeil étant située plus près d'une ligne à retard nul (ZDL) qu'une image de la choroïde de l'oeil, et dans lequel, dans le mode de fonctionnement choroïde (C), l'appareil de tomographie par cohérence optique (200) est configuré pour positionner une image de l'oeil dans l'orientation inversée (I), une image de la choroïde de l'oeil étant située plus près d'une ligne à retard nul (ZDL) qu'une image du corps vitré de l'oeil.

**2.** Procédé destiné à un appareil de tomographie par cohérence optique (200) selon la revendication 1, dans lequel l'étape d'observation consiste à observer une direction dans laquelle la rétine (127) s'est déplacée dans le tomogramme ou à observer si le déplacement test laisse, ou non, l'image de la rétine (127) à l'intérieur du tomogramme.

**3.** Procédé destiné à un appareil de tomographie par cohérence optique (200) selon la revendication 1 ou la revendication 2, dans lequel :
la position de l'image de la rétine (127) dans le tomogramme est déterminée par une mesure de la distance de l'image de la rétine (127) à partir d'une ligne à retard nul (ZDL) du tomogramme.

**4.** Procédé destiné à un appareil de tomographie par cohérence optique (200) selon la revendication 3, dans lequel le déplacement test (S121) consiste à :
sélectionner la première direction et la première distance conformément à :

$$CG_2 = CG_{PROG} + \alpha * R_{DEST}$$

où $CG_2$ est la position résultante de la ligne à retard nul (ZDL) après le déplacement test (S121) ; $CG_{PROG}$ est la position initiale de la ligne à retard nul (ZDL) ; $R_{DEST}$ est la position souhaitée de la rétine (127) à l'intérieur du tomogramme par rapport à la ligne à retard nul (ZDL) ; et $\alpha$ est un paramètre qui peut être positif de façon à augmenter la longueur de trajet optique de référence ou négatif de façon à réduire la longueur de trajet optique de référence.

**5.** Procédé destiné à un appareil de tomographie par cohérence optique (200) selon la revendication 3, dans lequel le déplacement test (S121) consiste à :
sélectionner la première direction et la première distance conformément à :

$$CG_2 = CG_{PROG} + w * P_{RET}$$

où $CG_2$ est la position résultante de la ligne à retard nul (ZDL) après le déplacement test (S121) ; $CG_{PROG}$ est la position initiale de la ligne à retard nul (ZDL) ; $P_{RET}$ est la position initiale de la rétine (127) à l'intérieur du tomogramme par rapport à la ligne à retard nul (ZDL) ; et w est un paramètre qui peut être positif de façon à augmenter la longueur de trajet optique de référence ou négatif de façon à réduire la longueur de trajet optique de référence.

**6.** Procédé destiné à un appareil de tomographie par cohérence optique (200) selon la revendication 3, dans lequel le déplacement test (S121) consiste à :
sélectionner la première direction et la première distance conformément à :

$$CG_2 = CG_{PROG} + m * (P_{RET} + R_{DEST})$$

où $CG_2$ est la position résultante de la ligne à retard nul (ZDL) après le déplacement test (S121) ; $CG_{PROG}$ est la position initiale de la ligne à retard nul (ZDL) ; $P_{RET}$ est la position initiale de la rétine (127) à l'intérieur du tomogramme par rapport à la ligne à retard nul (ZDL) ; $R_{DEST}$ est la position souhaitée de la rétine (127) à l'intérieur du tomogramme par rapport à la ligne à retard nul (ZDL) ; et m est un paramètre qui peut être positif de façon à augmenter la longueur de trajet optique de référence ou négatif de façon à réduire la longueur de trajet optique de référence.

**7.** Procédé destiné à un appareil de tomographie par cohérence optique (200) selon la revendication 3, dans lequel le déplacement test (S121) consiste à :
sélectionner la première direction et la première distance conformément à :

$$CG_2 = CG_{PROG} + w * \alpha * P_{RET}$$

où $CG_2$ est la position résultante de la ligne à retard nul (ZDL) après le déplacement test (S121) ; $CG_{PROG}$ est la position initiale de la ligne à retard nul (ZDL) ; $P_{RET}$ est la position initiale de la rétine (127) à l'intérieur du tomogramme par rapport à la ligne à retard nul (ZDL) ; $\alpha$ est un paramètre qui prend une valeur comprise entre $\alpha_{min} = | 1 - Z_{MAX}/(2*P_{RET}) | + Z_{MAX}/(2*P_{RET})$ et $\alpha_{max} = Z_{MAX}/P_{RET} + 1$, où $Z_{MAX}$ est une plage d'imagerie de l'appareil de tomographie par cohérence optique ; et w est un paramètre qui peut être positif de façon à augmenter la longueur de trajet optique de référence ou négatif de façon à réduire la longueur de trajet optique de référence.

8. Procédé destiné à un appareil de tomographie par cohérence optique (200) selon l'une quelconque des revendications 1 à 7, dans lequel, si la position de l'image de la rétine (127) à l'intérieur du tomogramme après le déplacement test correspond à une position souhaitée, il est alors déterminé qu'il n'est pas nécessaire d'effectuer le déplacement cible.

9. Procédé destiné à un appareil de tomographie par cohérence optique (200) selon l'une quelconque des revendications précédentes, dans lequel l'étape d'initialisation (S110) de la longueur de trajet optique de référence consiste à :
calculer une longueur de trajet optique de référence initiale qui est adaptée pour donner une orientation initiale de l'image de la rétine (127) à la position initiale, où l'orientation initiale est différente de l'orientation (T) correspondant au mode de fonctionnement défini.

10. Procédé destiné à un appareil de tomographie par cohérence optique (200) selon l'une quelconque des revendications précédentes, dans lequel l'étape d'initialisation (S110) de la longueur de trajet optique de référence comprend les étapes consistant à :

fixer la longueur de trajet optique de référence dans l'appareil de tomographie par cohérence optique (200) à une position limite de la plage de déplacement d'un miroir de référence (S113B) ; et
modifier la longueur de trajet optique de référence (S114B) jusqu'à ce que l'image de la rétine (127) soit visible sur le tomogramme (S115B).

11. Procédé destiné à un appareil de tomographie par cohérence optique (200) selon l'une quelconque des revendications 1 à 10, dans lequel :

l'objet est une rétine (127) d'un oeil (107) ; et
l'étape d'initialisation de la longueur de trajet optique de référence consiste à estimer une position de la rétine par une détermination d'une longueur axiale (D) de l'oeil (107) sur la base d'une erreur de réfraction (F) de l'oeil (107).

12. Procédé destiné à un appareil de tomographie par cohérence optique (200) selon l'une quelconque des revendications précédentes,
dans lequel l'estimation de la position de la rétine consiste à mesurer une position (w) d'un segment antérieur de l'oeil au moyen d'une lumière réfléchie à partir du segment antérieur de l'oeil et à ajouter la longueur axiale déterminée (D) de l'oeil mesurée au moyen de l'erreur de réfraction (F) de l'oeil qui a été mesurée au moyen d'un ophtalmoscope laser à balayage.

13. Procédé destiné à un appareil de tomographie par cohérence optique (200) selon l'une quelconque des revendications précédentes, comprenant en outre :

une étape ultérieure consistant à mesurer la position de l'image de la rétine (127) sur le tomogramme (S131) ;
comparer la position souhaitée ($R_{DEST}$) de l'image de la rétine (127) avec une position mesurée de l'image de la rétine (127) (S132) ; et
corriger la position de la rétine (127) par une modification de la longueur de trajet optique de référence de l'appareil de tomographie par cohérence optique (200) de façon à déplacer une image de la rétine (127) de la position mesurée à la position souhaitée (S134).

14. Procédé destiné à un appareil de tomographie par cohérence optique (200) selon l'une quelconque des revendications précédentes, comprenant en outre les étapes consistant à :

diviser le tomogramme en une pluralité de sections (1-7) ; et
déterminer la position de l'image de la rétine (127) par une identification d'une section du tomogramme dans laquelle est positionnée l'image de la rétine (127).

**15.** Procédé destiné à un appareil de tomographie par cohérence optique (200) selon la revendication 14, dans lequel la division du tomogramme en sections consiste à :

diviser le tomogramme en sections (1-7) de longueur égale,
où le procédé comprend en outre l'étape consistant à :
modifier la longueur de trajet optique en unités par rapport à la longueur des sections.

**16.** Procédé destiné à un appareil de tomographie par cohérence optique (200) selon l'une quelconque des revendications 1 à 15, dans lequel le déplacement cible (S123) est effectué sur la base du changement dans le tomogramme de la rétine (127) en tant que résultat du déplacement test, de la position initiale et de la position souhaitée de l'image de la rétine (127) à l'intérieur du tomogramme, et d'un mode de fonctionnement, dans lequel le mode de fonctionnement est l'un d'un mode de fonctionnement corps vitré (V) et d'un mode de fonctionnement choroïde (C).

**17.** Appareil de tomographie par cohérence optique (200) pour positionner, sur un tomogramme, une image d'une rétine (127) d'un l'oeil (107), l'appareil de tomographie par cohérence optique (200) comprenant :

un moyen d'initialisation (131-3) conçu pour initialiser une longueur de trajet optique de référence de l'appareil de tomographie par cohérence optique (200) de façon à pouvoir visualiser une image de la rétine (127) sur le tomogramme à une position initiale ($P_{RET}$), par une mise de l'appareil de tomographie par cohérence optique (200) dans l'un d'un mode de fonctionnement corps vitré et d'un mode de fonctionnement choroïde ;
un moyen de modification (132-4) configuré pour exécuter un déplacement test (S121) par une modification, d'une première distance, de la longueur de trajet optique de référence dans une première direction ;
un moyen d'observation (928) configuré pour observer un changement dans le tomogramme de la rétine (127) en tant que résultat du déplacement test; et
un moyen de détermination d'orientation (925) configuré pour déterminer, à partir de l'observation, si une orientation initiale de l'image de la rétine (127) est une orientation normale (N) ou une orientation inversée (I) ;
dans lequel le moyen de modification (132-4) est configuré pour effectuer un déplacement cible (S123) par une modification de la longueur de trajet optique de référence de l'appareil de tomographie par cohérence optique (200) de façon à pouvoir placer l'image de la rétine (127) à une position souhaitée à l'intérieur du tomogramme sur la base de l'orientation initiale de l'image de la rétine (127) ;
dans lequel, dans le mode de fonctionnement corps vitré (V), l'appareil de tomographie par cohérence optique (200) est configuré pour positionner une image de l'oeil dans l'orientation normale (N), une image du corps vitré de l'oeil étant située plus près d'une ligne à retard nul (ZDL) qu'une image de la choroïde de l'oeil, et
dans lequel, dans le mode de fonctionnement choroïde (C), l'appareil de tomographie par cohérence optique (200) est configuré pour positionner une image de l'oeil dans l'orientation inversée (I), une image de la choroïde de l'oeil étant située plus près d'une ligne à retard nul (ZDL) qu'une image du corps vitré de l'oeil.

**18.** Appareil de tomographie par cohérence optique (200) selon la revendication 17, comprenant en outre :

un trajet optique côté objet (S) entre la rétine (127) et un interféromètre ; et
un trajet optique côté référence (R) entre un miroir de référence (132-4) et l'interféromètre;
dans lequel la longueur de trajet optique de référence qui est réglée est une longueur de trajet optique du trajet optique côté référence (R).

**19.** Appareil de tomographie par cohérence optique (200) selon la revendication 17, comprenant en outre :

un trajet optique côté objet (S) entre la rétine (127) et un interféromètre ; et
un trajet optique côté référence (R) entre un miroir de référence (132-4) et l'interféromètre ;
dans lequel la longueur de trajet optique de référence qui est réglée est une longueur de trajet optique du trajet optique côté objet (S).

**20.** Appareil de tomographie par cohérence optique (200) selon l'une quelconque des revendications 17 à 19, comprenant en outre :

une source de lumière (101) destinée à émettre une lumière de faible cohérence ;

un interféromètre destiné à générer une lumière d'interférence par une division de la lumière de faible cohérence émise en une lumière d'échantillon à transmettre dans un trajet d'échantillon (S) vers une rétine (127) à mesurer et en une lumière de référence à transmettre dans un trajet de référence (R) vers un objet de référence, et à superposer la lumière d'échantillon ayant traversé la rétine (127) à mesurer et la lumière de référence ayant traversé l'objet de référence,

un détecteur destiné à détecter la lumière d'interférence générée ; et

un moyen de formation d'image destiné à former une image de la rétine (127) à mesurer sur un tomogramme sur la base du résultat de détection du détecteur,

dans lequel le moyen de modification (132-4) est configuré pour modifier une différence de longueur de trajet optique entre la lumière d'échantillon et la lumière de référence sur la base du résultat de détermination du moyen de détermination d'orientation (925).

21. Appareil de tomographie par cohérence optique (200) selon l'une quelconque des revendications 17 à 20, dans lequel le déplacement cible (S123) est effectué sur la base du changement dans le tomogramme de la rétine (127) en tant que résultat du déplacement test, de la position initiale et de la position souhaitée de l'image de la rétine (127) à l'intérieur du tomogramme, et d'un mode de fonctionnement,

dans lequel le mode de fonctionnement est l'un d'un mode de fonctionnement corps vitré (V) et d'un mode de fonctionnement choroïde (C).

22. Programme qui, lorsqu'il est implémenté dans un appareil de tomographie par cohérence optique (200), amène l'appareil de tomographie par cohérence optique (200) à mettre en oeuvre un procédé selon l'une quelconque des revendications 1 à 16.

23. Support d'informations contenant en mémoire un programme selon la revendication 22.

Fig. 1A

Fig. 1B

925

401
IMAGE
ACQUIRING
PORTION

402
SCAN
CONTROL
PORTION

403
IMAGE
FORMING
PORTION

404
3D IMAGE
FORMING
PORTION

405
3D IMAGE
EXTRACTING
PORTION

IMAGE PROCESSING
PORTION

406
DISPLAY
CONTROL
PORTION

Fig. 2

Fig. 3A

Fig. 3B

C

**CHOROID MODE**

N

VITREUM

ZDL

ZERO-DELAY
LINE

CHOROIDEA

CHOROIDEA

I

VITREUM

THIS PART OF IMAGE IS ANALYZED

V

**VITREOUS MODE**

CHOROIDEA

I

VITREUM

ZERO-DELAY
LINE

N

VITREUM

CHOROIDEA

THIS PART OF IMAGE IS ANALYZED

-Z max

0

SENSITIVITY

POSITION

+Z max

Fig. 4A          Fig. 4B          Fig. 4C

Fig. 5

S110

S111A

Start

Determine head position

S112A

Set focus

S113A

Calculate axial length of eye

S114A

Move mirror

S115A

Object visible on tomogram?   NO

YES

A

Fig. 6A

S110

S111B

Start

Determine head position

S112B

Set focus

S113B

Set mirror at limit position

S114B

Start moving mirror

S115B

Object visible on tomogram?    NO

S116B

YES

Stop moving mirror

A

Fig. 6B

S120

S121

Test move

S122

Desired view?

YES

S123

NO

Target move

B

A

Fig. 7

S130

S131

S132

S133

S134

```
           ┌───┐
           │ B │
           └───┘
             │
             ▼
┌──────────────────────────────┐
│  Measure position of object  │
└──────────────────────────────┘
             │
             ▼
┌──────────────────────────────┐
│   Calculate position error   │
└──────────────────────────────┘
             │
             ▼
        ╱─────────╲                YES
       ╱ Is error < ╲──────────────────┐
       ╲ precision? ╱                  │
        ╲─────────╱                    │
             │ NO                      │
             ▼                         │
┌──────────────────────────────┐       │
│   Correct object position    │       │
└──────────────────────────────┘       │
             │                         │
             ▼                         │
          ╭───────╮                    │
          │  End  │◄───────────────────┘
          ╰───────╯
```

Fig. 8

Fig. 9

Fig. 10A

Fig. 10B

ZDL

ZDL

T

I

I

T

N

ZDL

ZDL

N

T

N

T

ZDL

ZDL

I

N

I

N

Z

Z

Fig. 10C

Fig. 10D

w = -1, m = -1

w = -1, m = -1

ZDL

N          T          I

ZDL

I          T          N

ZDL

I          N

ZDL

T

N

ZDL

I          N

Z

Z

Vitreous
Mode (V)

Fig. 11A

Fig. 11B

$+P\_RET$

$-R\_DEST$

$+z$

Choroid
Mode (C)

Fig. 11C

Fig. 11D

$-P\_RET$

$-R\_DEST$

Vitreous
Mode (V)

I

N

$+P\_RET$

$+R\_DEST$

$+z$

Fig. 12A

Fig. 12B

Choroid
Mode (C)

N

I

$-P\_RET$

$+R\_DEST$

N

I

Fig. 12C

Fig. 12D

Vitreous
Mode (V)

+P_RET

Fig. 13A

+P_RET

+z

Fig. 13B

Choroid
Mode (C)

-P_RET

+P_RET

N&I

Fig. 13C

Fig. 13D

Vitreous
Mode (V)

Fig. 14A
Fig. 14B

Choroid
Mode (C)

Fig. 14C
Fig. 14D

## Fig. 15A

After init position
CG = $CG_{PROG}$

After test move
CG = $CG_2$

v = 1

$TM = -5/3\ P_{RET}$

After target move
CG = $CG_{DEST}$

$CG_{DEST} = CG_2 + 2/3 P_{RET} - R_{DEST}$

## Fig. 15B

After init position
$CG = CG_{PROG}$

After test move
$CG = CG_2$

$v = -1$

$TM = -5/3\ P_{RET}$

After target move
$CG = CG_{DEST}$

$CG_{DEST} = CG_2 + 8/3P_{RET} - R_{DEST}$

Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100014089 A
- US 7643154 B2
- US 7777893 B2
- US 7982880 B2
- US 20130182218 A1
- US 20110181889 A
- US 20090244547 A
- US 20130182218 A
- PL 218325 B1
- US 6111645 A
- EP 2702933 A1